# EUROPÄISCHE PATENTSCHRIFT

(11) **EP 1 513 841 B1**
(45) Veröffentlichungstag und Bekanntmachung des Hinweises auf die Patenterteilung: **14.05.2008**
(21) Anmeldenummer: 03735536.9
(22) Anmeldetag: 04.06.2003
(51) Int. Cl.: C07D 471/06, C09B 5/62

(54) **1,6,9,14-TETRASUBSTITUIERTE TERRYLENTETRACARBONS UREDIIMIDE**
1,6,9,14-TETRASUBSTITUTED TERRYLENE TETRACARBOXYLIC ACID DIIMIDES
DIIMIDE D'ACIDE T TRACARBONIQUE TERRYLENE 1,6,9,14-T TRASUBSTITUE

(30) Priorität: 07.06.2002 DE 10225595
(43) Veröffentlichungstag der Anmeldung: 16.03.2005
(73) Patentinhaber: BASF SE, 67056 Ludwigshafen (DE); Max-Planck-Gesellschaft zur Förderung der Wissenschaften e.V., Berlin (DE)
(72) Erfinder: BÖHM, Arno, 68305 Mannheim (DE); KRIEGER, Matthias, 79538 Lörrach (DE); REUTHER, Erik, 55122 Mainz (DE); MÜLLEN, Klaus, 50939 Köln (DE)
(74) Vertreter: Isenbruck, Günter
(86) Internationale Anmeldenummer: PCT/EP2003/005817
(87) Internationale Veröffentlichungsnummer: WO 2003/104232

(56) Entgegenhaltungen:
- WO-A-02/076988
- HOLTRUP F O ET AL: "TERRYLENIMIDES: NEW NIR FLUORESCENT DYES" CHEMISTRY - A EUROPEAN JOURNAL, VCH PUBLISHERS, US, Bd. 3, Nr. 2, 1997, Seiten 219-225, XP000931226 ISSN: 0947-6539

## Beschreibung

Die vorliegende Erfindung betrifft neue 1,6,9,14-tetrasubstituierte Terrylentetracarbonsäurediimide der allgemeinen Formel I 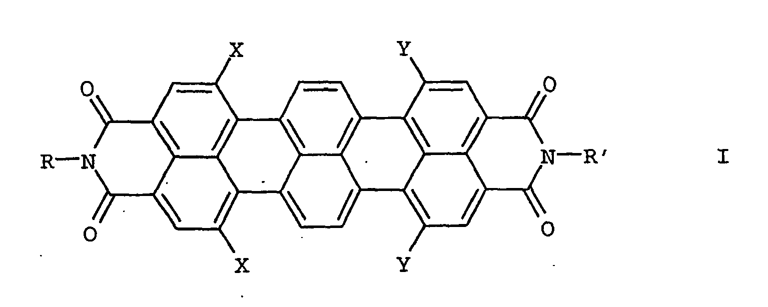 in der die Variablen folgende Bedeutung haben:
- X, Y: unabhängig voneinander
Brom; Cyano;
Aryloxy, Arylthio, Hetaryloxy oder Hetarylthio, das jeweils ein- oder mehrfach durch C₁-C₁₂-Alkyl, C₁-C₁₂-Alkoxy, -COOR¹, -SO₃R¹, Halogen, Hydroxy, Carboxy, Cyano, -CONHR² und/oder -NHCOR² substituiert sein kann;
einen Rest der Formel -L-R³;
einen Rest der Formel -NR²₂;
- R, R': unabhängig voneinander
Wasserstoff;
C₁-C₃₀-Alkyl, dessen Kohlenstoffkette durch eine oder mehrere Gruppierungen -O-, -S-, -NR¹-, -CO- und/oder -SO₂- unterbrochen sein kann und das durch Cyano, C₁-C₆-Alkoxy, Aryl, das durch C₁-C₁₈-Alkyl oder C₁-C₆-Alkoxy substituiert sein kann, und/oder einen über ein Stickstoffatom gebundenen 5- bis 7-gliedrigen heterocyclischen Rest, der weitere Heteroatome enthalten und aromatisch sein kann, ein- oder mehrfach substituiert sein kann;
C₅-C₈-Cycloalkyl, dessen Kohlenstoffgerüst durch eine oder mehrere Gruppierungen -O-, -S- und/oder -NR¹- unterbrochen und/oder das durch C₁-C₆-Alkyl ein- oder mehrfach substituiert sein kann;
Aryl oder Hetaryl, das durch C₁-C₁₈-Alkyl, C₁-C₆-Alkoxy, Cyano, -CONHR², -NHCOR² und/oder Aryl- oder Hetarylazo, das jeweils durch C₁-C₁₀-Alkyl, C₁-C₆-Alkoxy oder Cyano substituiert sein kann, ein- oder mehrfach substituiert sein kann;
- L: 1,2-Ethylen, 1,2-Ethenylen oder 1,2-Ethinylen;
- R¹: Wasserstoff oder C₁-C₆-Alkyl;
- R²: Wasserstoff; C₁-C₁₈-Alkyl; Aryl oder Hetaryl, das jeweils durch C₁-C₆-Alkyl, C₁-C₆-Alkoxy, Halogen, Hydroxy, Carboxy oder Cyano substituiert sein kann;
- R³: C₁-C₁₈-Alkyl, dessen Kohlenstoffkette durch eine oder mehrere Gruppierungen -O-, -S-, -NR¹-, -CO- und/oder -SO₂-unterbrochen sein kann und das durch -COOR¹, -SO₃R¹, Hydroxy, Cyano, C₁-C₆-Alkoxy, Aryl, das durch C₁-C₁₈-Alkyl oder C₁-C₆-Alkoxy substituiert sein kann, und/oder einen über ein Stickstoffatom gebundenen 5- bis 7-gliedrigen heterocyclischen Rest, der weitere Heteroatome enthalten und aromatisch sein kann, ein- oder mehrfach substituiert sein kann,
mit der Maßgabe, dass N,N'-Bis(2,6-diisopropylphenyl)-1,6,9,14-tetrabxomterrylen-3,4:11,12-tetracarbonsäurediimid, N,N'-Bis(2,6-diisopropylphenyl)-1,6,9,14-tetra(p-tert.-octylphenoxy)terrylen-3,4:11,12-tetracarbonsäurediimid, N-Cyclohexyl-N'-(2,6-diisopropylphenyl)-1,6,9,14-tetrabromterrylen-3,4:11,12-tetracarbonsäurediimid, N-Cyclohexyl-N'-(2,6-diisopropylphenyl)-1,6,9,14-tetra(p-tert.-octylphenoxy)terrylen-3,4:11,12-tetracarbonsäurediimid ausgeschlossen sind,
sowie die Herstellung dieser Terrylentetracarbonsäurediimide und ihre Verwendung zur Einfärbung von hochmolekularen organischen und anorganischen Materialien, als Dispergierhilfsmittel, Pigmentadditive für organische Pigmente und zwischenprodukte für die Herstellung von Fluoreszenzfarbmitteln und Pigmentadditiven, zur Herstellung farbiger oder im nahinfraroten Bereich des elektromagnetischen Spektrums absorbierender und/oder emittierender wäßriger Polymerisatdispersionen, als Photoleiter in der Elektrophotographie, als Emitter in Elektro- und Chemilumineszenzanwendungen, als Aktivkomponenten in der Fluoreszenzkonversion, in Biolumineszenzarrays sowie der Photovoltaik und als Laserfarbstoffe.

Wie aus J. Phys. Chem. A 1997, 101, S. 8435-8440 sowie Chem. Eur. J. 1997, 3, S. 219-225 bekannt, eignen sich Terrylen-3,4:11,12-tetracarbonsäurediimide (im folgenden kurz "Terrylimide" genannt) vorteilhaft als Pigmente und Fluoreszenzfarbstoffe mit Absorption im langwellig roten und Fluoreszenzemission im langwellig roten bis nahinfraroten Bereich des elektromagnetischen Spektrums.

Im einzelnen wird in Chem. Eur. J. 1997, 3, S. 219-225 die Herstellung von nichtkernsubstituierten und 1,6-di-tert.-butylphenoxysubstituierten Terrylimiden beschrieben.

Während die am Terrylenkern unsubstituierten Vertreter dieser Substanzklasse über die beschriebene Synthesesequenz in befriedigenden Ausbeuten zugänglich sind, ist eine Darstellung der 1,6-disubstituierten Derivate synthetisch äußerst aufwendig und liefert die gewünschten Produkte nur in unbefriedigenden Ausbeuten. Eine Darstellung höher substituierter Derivate mit längerwelliger Absorption und Emission ist über die bekannte Route prinzipiell nicht möglich.

In der nicht vorveröffentlichten WO 02/076988 werden N,N'-Bis(2,6-diisopropylphenyl)-1,6,9,14-tetrabromterrylen-3,4:11,12-tetracarbonsäurediimid, N,N'-Bis(2,6-diisopropylphenyl)-1,6,9,14-tetra(p-tert.-octylphenoxy)terrylen-3,4:11,12-tetracarbonsäurediimid, N-Cyclohexyl-N'-(2,6-diisopropylphenyl)-1,6,9,14-tetrabromterrylen-3;4:11,12-tetracarbonsäurediimid, N-Cyclohexyl-N'-(2,6-diisopropylphenyl)-1,6,9,14-tetra(p-tert.-octylphenoxy)terrylen-3,4:11,12-tetracarbonsäurediimid offenbart.

Der Erfindung lag daher die Aufgabe zugrunde, höher substituierte Terrylimide mit vorteilhaften Anwendungseigenschaften, die insbesondere nicht nur gut in das jeweilige Anwendungsmedium einarbeitbar und an dieses Medium anpaßbar sind, sondern auch langwelliger als die bisher bekannten Vertreter dieser Substanzklasse, d.h. im langwellig roten und nahinfraroten Bereich des elektromagnetischen Spektrums, absorbieren und emittieren, bereitzustellen.

Demgemäß wurden die eingangs definierten Terrylimide der Formel I gefunden.

Bevorzugte Terrylimide sind dem Unteranspruch zu entnehmen.

Weiterhin wurde ein Verfahren zur Herstellung von 1,6,9,14-tetrasubstituierten Terrylimiden der allgemeinen Formel Ia 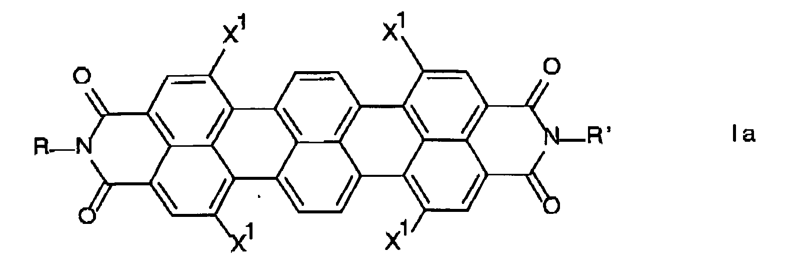 in der X¹ Brom; Aryloxy, Arylthio, Hetaryloxy oder Hetarylthio, das jeweils ein- oder mehrfach durch C₁-C₁₂-Alkyl, C₁-C₁₂-Alkoxy, - COOR¹, -SO₃R¹, Halogen, Hydroxy, Carboxy, Cyano, -CONHR² und/oder -NHCOR² substituiert sein kann, bedeutet, mit der Maßgabe, dass N,N'-Bis(2,6-diisopropylphenyl)-1,6,9,14-tetrabromterrylen-3,4:11,12-tetracarbonsäurediimid, N,N'-Bis(2,6-diisopropylphenyl)-1,6,9,14-tetra(p-tert.-octylphenoxy)terrylen-3,4:11,12-tetracarbonsäurediimid, N-Cyclohexyl-N'-(2,6-diisopropylphenyl)-1,6,9,14-tetrabromterrylen-3,4:11,12-tetracarbonsäurediimid, N-Cyclohexyl-N'-(2,6-diisopropylphenyl)-1,6,9,14-tetra(p-tert.-octylphenoxy)terrylen-3,4:11,12-tetracarbonsäurediimid ausgeschlossen sind, gefunden, welches dadurch gekennzeichnet ist, dass man
a) ein Terrylimid der allgemeinen Formel II 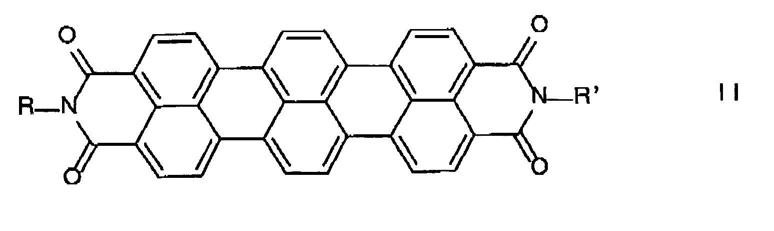 mit elementarem Brom in Gegenwart eines Halogenkohlenwasserstoffs als Lösungsmittel zum 1, 6, 9, 14-Tetrabromterrylimid der allgemeinen Formel Ia' 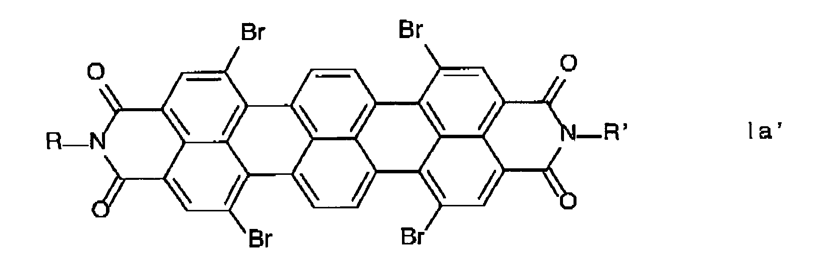 umsetzt und gewünschtenfalls
b) das in Schritt a) erhaltene Tetrabromterrylimid Ia' in Gegenwart eines inerten stickstoffbasischen Lösungsmittels und einer Base mit einem Alkohol oder Thioalkohol der allgemeinen Formel III mit X¹ ≠ Brom 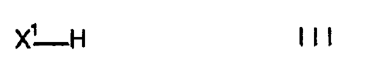 in das 1,6,9,14-tetrasubstituierte Terrylimid der Formel Ia mit X¹ ≠ Brom mit der Maßgabe, dass N,N'-Bis(2,6-diisopropylphenyl)-1,6,9,4-tetra(p-tert.-octylphenyl)-terrylen-3,4;11,12-dicarbonsäurediimid und N-Cyclohexyl-N'-(2,6-diisopropylphenyl)-1,6,9,14-tetra-(p-tert.-octylphenoxy)terrylen-3,4:11,12-dicarbonsäurediimid ausgenommen sind, überführt.

Außerdem wurde ein Verfahren zur Herstellung von 1,6,9,14-tetrasubstituierten Terrylimiden der allgemeinen Formel Ib 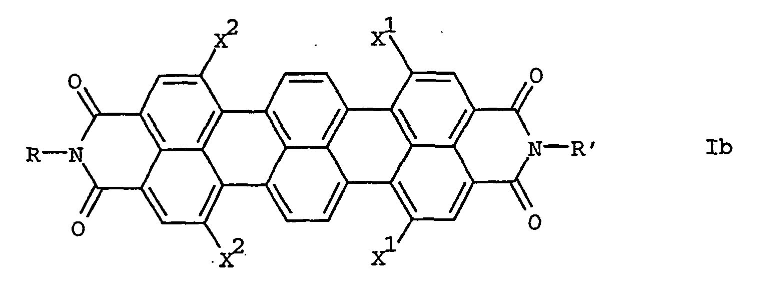 in der
- X²: Aryloxy, Arylthio, Hetaryloxy oder Hetarylthio, das jeweils ein- oder mehrfach durch C₁-C₁₂-Alkyl, C₁-C₁₂-Alkoxy, -COOR¹, -SO₃R¹, Cyano, -CONHR⁴ und/oder -NHCOR⁴ substituiert sein kann,
- Y²: Brom; Aryloxy, Arylthio, Hetaryloxy oder Hetarylthio, das jeweils ein- oder mehrfach durch C₁-C₁₂-Alkyl, C₁-C₁₂-Alkoxy, -COOR¹, -SO₃R^{l}, Halogen, Hydroxy, Carboxy, Cyano, -CONHR² und/oder -NHCOR² substituiert sein kann, und
- R⁴: C₁-C₁₈-Alkyl; Aryl oder Hetaryl, das jeweils durch C₁-C₆-Alkyl, C₁-C₆-Alkoxy, Hydroxy oder Cyano substituiert sein kann, bedeuten,
gefunden, welches dadurch gekennzeichnet ist, daß man
a) ein 1,6-disubstiutiertes Terrylimid der allgemeinen Formel IV 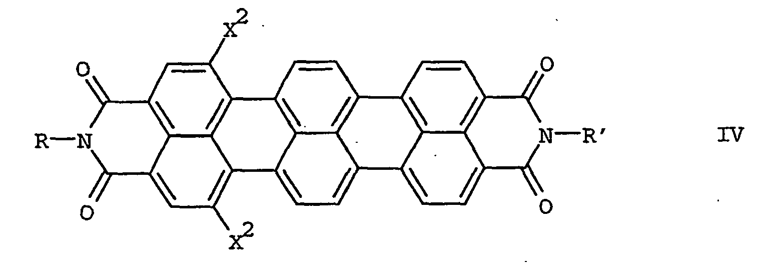 mit elementarem Brom in Gegenwart eines Halogenkohlenwasserstoffs als Lösungsmittel zum 9,14-dibromierten Terrylimid der allgemeinen Formel Ib' 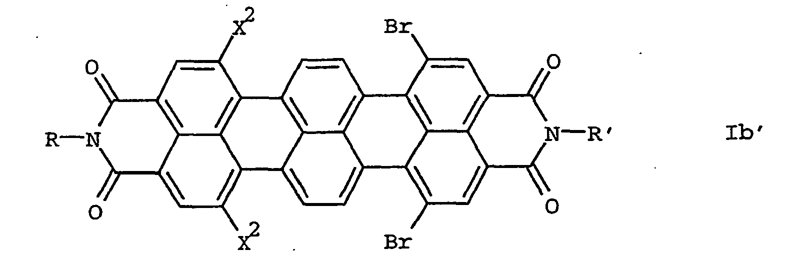 umsetzt und gewünschtenfalls
b) das in Schritt a) erhaltene 9,14-dibromierte Terrylimid Ib' in Gegenwart eines inerten stickstoffbasischen Lösungsmittels und einer Base mit einem Alkohol oder Thioalkohol der allgemeinen Formel III mit X¹ ≠ Brom 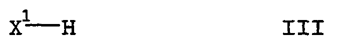 in das 1,6,9,14-tetrasubstituierte Terrylimid der Formel Ib mit X¹ ≠ Brom überführt.

Weiterhin wurde ein Verfahren zur Herstellung von 1,6,9,14-tetrasubstituierten Terrylimiden der allgemeinen Formel Ic 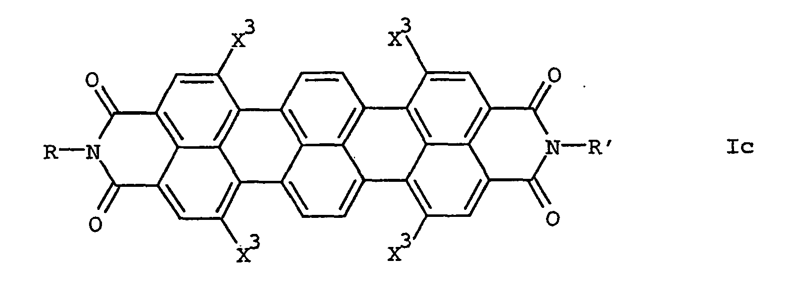 in der X³ einen Rest der Formel -L-R³ bedeutet, gefunden, welches dadurch gekennzeichnet ist, daß man ein 1,6,9,14-Tetrabromterrylimid der allgemeinen Formel Ia' 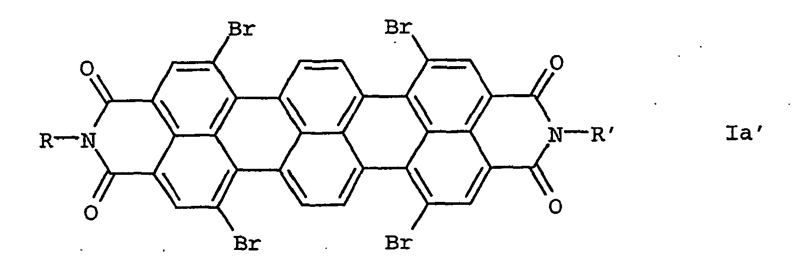 in Gegenwart eines aprotischen Lösungsmittels, eines Palladiumkomplexes als Katalysator, eines Kupfersalzes als Cokatalysator und einer Base mit einem 1-Alkin der allgemeinen Formel V
H-C≡C-R³ V
umsetzt und gewünschtenfalls die in den Resten X³ des erhaltenen 1,6,9,14-tetrasubstituierten Terrylimids enthaltenen ungesättigten Bindungen zusätzlich reduziert.

Schließlich wurde ein Verfahren zur Herstellung von 1,6,9,14-tetrasubstituierten Terrylimiden der allgemeinen Formel Id 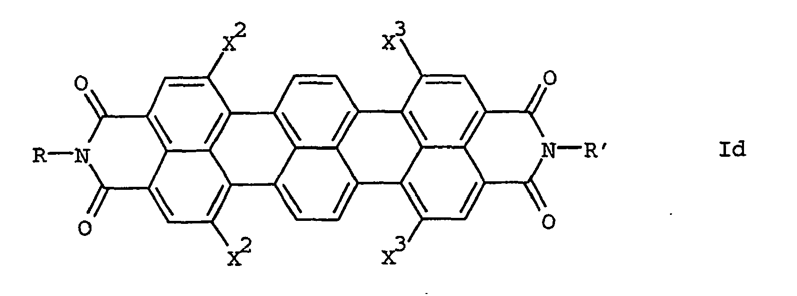 in der
- X²: Aryloxy, Arylthio, Hetaryloxy oder Hetarylthio, das jeweils ein- oder mehrfach durch C₁-C₁₂-Alkyl C₁-C₁₂-Alkoxy, -COOR¹, -SO₃R¹, Cyano, -CONHR⁴ und/oder -NHCOR⁴ substituiert sein kann,
- X³: einen Rest der Formel -L-R³ und
- R⁴: C₁-C₁₈-Alkyl; Aryl oder Hetaryl, das jeweils durch C₁-C₆-Alkyl, C₁-C₆-Alkoxy, Hydroxy oder Cyano substituiert sein kann, bedeuten,
gefunden, welches dadurch gekennzeichnet ist, daß man ein 9,14-dibromiertes Terrylimid der allgemeinen Formel Ib' in Gegenwart eines aprotischen Lösungsmittels, eines Palladiumkomplexes als Katalysator, eines Kupfersalzes als Cokatalysator und einer Base mit einem 1-Alkin der allgemeinen Formel V

H-C≡C-R³ V

umsetzt und gewünschtenfalls die in den Resten X³ des erhaltenen 1,6,9,14-tetrasubstituierten Terrylimids enthaltenen ungesättigten Bindungen zusätzlich reduziert.

Nicht zuletzt wurde die Verwendung der Terrylimide I zur Einfärbung von hochmolekularen organischen und anorganischen Materialien, als Dispergierhilfsmittel, Pigmentadditive für organische Pigmente und Zwischenprodukte für die Herstellung von Fluoreszenzfarbmitteln und Pigmentadditiven, zur Herstellung farbiger oder im nahinfraroten Bereich des elektromagnetischen Spektrums absorbierender und/oder emittierender wäßriger Polymerisatdispersionen, als Photoleiter in der Elektrophotographie, als Emitter in Elektro- und Chemilumineszenzanwendungen, als Aktivkomponenten in der Fluoreszenzkonversion, in Biolumineszenzarrays sowie der Photovoltaik und als Laserfarbstoffe gefunden.

Alle in den Formeln I bis V auftretenden Alkylgruppen können geradkettig oder verzweigt sein. Wenn die Alkylgruppen substituiert sind, tragen sie in der Regel 1 oder 2 Substituenten.

Aromatische Reste, die substituiert sind, können im allgemeinen bis zu 3, bevorzugt 1 oder 2, der genannten Substituenten aufweisen.

Als bevorzugte Reste -L-R³ sind durch C₁-C₁₈-Alkyl, vor allem C₄-C₈-Alkyl, das insbesondere endständig (ω-Position) durch Cyano, Hydroxy, Carboxy, Methyl- oder Ethylcarboxy substituiert sein kann, substitutiertes Ethenyl und Ethinyl zu nennen.

Als Beispiele für geeignete Reste R, R', R¹, R², R³, R⁴, X, X¹, X², X³, Y und Y² (bzw. für deren Substituenten) sowie -L-R³ seien im einzelnen genannt:
Methyl, Ethyl, Propyl, Isopropyl, Butyl, Isobutyl, tert.-Butyl, Pentyl, Isopentyl, Neopentyl, tert.-Pentyl, Hexyl, 2-Methylpentyl, Heptyl, 1-Ethylpentyl, Octyl, 2-Ethylhexyl, Isooctyl, Nonyl, Isononyl, Decyl, Isodecyl, Undecyl, Dodecyl, Tridecyl, Isotridecyl, Tetradecyl, Pentadecyl, Hexadecyl, Heptadecyl, Octadecyl, Nonadecyl und Eicosyl (die obigen Bezeichnungen Isooctyl, Isononyl, Isodecyl und Isotridecyl sind Trivialbezeichnungen und
   stammen von den nach der Oxosynthese erhaltenen Alkoholen);
Methoxymethyl, 2-Methoxyethyl, 2-Ethoxyethyl, 2-Propoxyethyl, 2-Isopropoxyethyl, 2-Butoxyethyl, 2- und 3-Methoxypropyl, 2- und 3-Ethoxypropyl, 2- und 3-Propoxypropyl, 2- und 3-Butoxypropyl, 2-und 4-Methoxybutyl, 2- und 4-Ethoxybutyl, 2- und 4-Propoxybutyl, 3,6-Dioxaheptyl, 3,6-Dioxaoctyl, 4,8-Dioxanonyl, 3,7-Dioxaoctyl, 3,7-Dioxanonyl, 4,7-Dioxaoctyl, 4,7-Dioxanonyl, 2- und 4-Butoxybutyl, 4,8-Dioxadecyl, 3,6,9-Trioxadecyl, 3,6,9-Trioxaundecyl, 3,6,9-Trioxadodecyl, 3,6,9,12-Tetraoxatridecyl und 3,6,9,12-Tetraoxatetradecyl;
Methylthiomethyl, 2-Methylthioethyl, 2-Ethylthioethyl, 2-Propylthioethyl, 2-Isopropylthioethyl, 2-Butylthioethyl, 2- und 3-Methylthiopropyl, 2- und 3-Ethylthiopropyl, 2- und 3-Propylthiopropyl, 2- und 3-Butylthiopropyl, 2- und 4-Methylthiobutyl, 2- und 4-Ethylthiobutyl, 2- und 4-Propylthiobutyl, 3,6-Dithiaheptyl, 3,6-Dithiaoctyl, 4,8-Dithianonyl, 3,7-Dithiaoctyl, 3,7-Dithianonyl, 2- und 4-Butylthiobutyl, 4,8-Dithiadecyl, 3,6,9-Trithiadecyl, 3,6,9-Trithiaundecyl, 3,6,9-Trithiadodecyl, 3,6,9,12-Tetrathiatridecyl und 3,6,9,12-Tetrathiatetradecyl;
2-Monomethyl- und 2-Monoethylaminoethyl, 2-Dimethylaminoethyl, 2-und 3- Dimethylaminopropyl, 3-Monoisopropylaminopropyl, 2- und 4-Monopropylaminobutyl, 2- und 4-Dimethylaminobutyl, 6-Methyl-3,6-diazaheptyl, 3,6-Dimethyl-3,6-diazaheptyl, 3,6-Diazaoctyl, 3,6-Dimethyl-3,6-diazaoctyl, 9-Methyl-3,6,9-triazadecyl, 3,6,9-Trimethyl-3,6,9-triazadecyl, 3,6,9-Triazaundecyl, 3,6,9-Trimethyl-3,6,9-triazaundecyl, 12-Methyl-3,6,9,12-tetraazatridecyl und 3,6,9,12-Tetramethyl-3,6,9,12-tetraazatridecyl;
Propan-2-on-1-yl, Butan-3-on-1-yl, Butan-3-on-2-yl und 2-Ethylpentan-3-on-1-yl;
2-Methylsulfonylethyl, 2-Ethylsulfonylethyl, 2-Propylsulfonylethyl, 2-Isopropylsulfonylethyl, 2-Butylsulfonylethyl, 2- und 3-Methylsulfonylpropyl, 2- und 3-Ethylsulfonylpropyl, 2- und 3-Propylsulfonylpropyl, 2- und 3-Butylsulfonylpropyl, 2- und 4-Methylsulfonylbutyl, 2- und 4-Ethylsulfonylbutyl, 2- und 4-Propylsulfonylbutyl und 4-Butylsulfonylbutyl;
Carboxymethyl, 2-Carboxyethyl, 3-Carboxypropyl, 4-Carboxybutyl, 5-Carboxypentyl, 6-Carboxyhexyl, 8-Carboxyoctyl, 10-Carboxydecyl, 12-Carboxydodecyl und 14-Carboxytetradecyl;
Sulfomethyl, 2-Sulfoethyl, 3-Sulfopropyl, 4-Sulfobutyl, 5-Sulfopentyl, 6-Sulfohexyl, 8-Sulfooctyl, 10-Sulfodecyl, 12-Sulfododecyl und 14-Sulfotetradecyl;
2-Hydroxyethyl, 3-Hydroxypropyl, 1-Hydroxyprop-2-yl, 2- und 4-Hydroxybutyl, 1-Hydroxybut-2-yl und 8-Hydroxy-4-oxaoctyl;
Cyanomethyl, 2-Cyanoethyl, 3-Cyanopropyl, 2-Methyl-3-ethyl-3-cyanopropyl, 7-Cyano-7-ethylheptyl und 4,7-Dimethyl-7-cyanoheptyl;
Methoxy, Ethoxy, Propoxy, Isopropoxy, Butoxy, Isobutoxy, sec.-Butoxy, tert.-Butoxy, Pentoxy, Isopentoxy, Neopentoxy, tert.-Pentoxy und Hexoxy;
Carbamoyl, Methylaminocarbonyl, Ethylaminocarbonyl, Propylaminocarbonyl, Butylaminocarbonyl, Pentylaminocarbonyl, Hexylaminocarbonyl, Heptylaminocarbonyl, Octylaminocarbonyl, Nonylaminocarbonyl, Decylaminocarbonyl und Phenylaminocarbonyl;
Formylamino, Acetylamino, Propionylamino und Benzoylamino;
Chlor, Brom und Iod;
Phenylazo, 2-Napthylazo, 2-Pyridylazo und 2-Pyrimidylazo;
Phenyl, 1- und 2-Naphthyl, 2- und 3-Pyrryl, 2-, 3- und 4-Pyridyl, 2-, 4- und 5-Pyrimidyl, 3-, 4- und 5-Pyrazolyl, 2-, 4- und 5-Imidazolyl, 2-, 4- und 5-Thiazolyl, 3-(1,2,4-Triazyl), 2-(1,3,5-Triazyl), 6-Chinaldyl, 3-, 5-, 6- und 8-Chinolinyl, 2-Benzoxazolyl, 2-Benzothiazolyl, 5-Benzothiadiazolyl, 2- und 5-Benzimidazolyl und 1- und 5- Isochinolyl;
2-, 3- und 4-Methylphenyl, 2,3-, 2,4-, 2,5-, 3,5- und 2,6-Dimethylphenyl, 2,4,6-Trimethylphenyl, 2-, 3- und 4-Ethylphenyl, 2,3-, 2,4-, 2,5-, 3,5- und 2,6-Diethylphenyl, 2,4,6-Triethylphenyl, 2-, 3- und 4-Propylphenyl, 2,3-, 2,4-, 2,5-, 3,5-und 2,6-Dipropylphenyl, 2,4,6-Tripropylphenyl, 2-, 3- und 4-Isopropylphenyl, 2,3-, 2,4-, 2,5-, 3,5- und 2,6-Diisopropylphenyl, 2,4,6-Triisopropylphenyl, 2-, 3- und 4-Butylphenyl, 2,3-, 2,4-, 2,5-, 3,5- und 2,6-Dibutylphenyl, 2,4,6-Tributylphenyl, 2-, 3-und 4-Isobutylphenyl, 2,3-, 2,4-, 2,5-, 3,5- und 2,6-Diisobutylphenyl, 2,4,6-Triisobutylphenyl, 2-, 3- und 4-sec.-Butylphenyl, 2,3-, 2,4-, 2,5-, 3,5- und 2,6-Di-sec.-butylphenyl und 2,4,6-Tri-sec.-butylphenyl, 2-, 3- und 4-tert.-Butylphenyl, 2,3-, 2,4-, 2,5-, 3,5- und 2,6-Di-tert.-butylphenyl, 2,4,6-Tri-tert.-butylphenyl; 2-, 3- und 4-Methoxyphenyl, 2,3-, 2,4-, 2,5-, 3,5- und 2,6-Dimethoxyphenyl, 2,4,6-Trimethoxyphenyl, 2-, 3- und 4-Ethoxyphenyl, 2,3-, 2,4-, 2,5-, 3,5- und 2,6-Diethoxyphenyl, 2,4,6-Triethoxyphenyl, 2-, 3- und 4-Propoxyphenyl, 2,3-, 2,4-, 2,5-, 3,5-und 2,6-Dipropoxyphenyl, 2-, 3- und 4-Isopropoxyphenyl, 2,3-, 2,4-, 2,5-, 3,5- und 2,6-Diisopropoxyphenyl und 2-, 3- und 4-Butoxyphenyl; 2-, 3- und 4-Chlorphenyl, und 2,3-, 2,4-, 2,5-, 3,5- und 2,6-Dichlorphenyl; 2-, 3- und 4-Hydroxyphenyl und 2,3-, 2,4-, 2,5-, 3,5- und 2,6-Dihydroxyphenyl; 2-, 3- und 4-Cyanophenyl; 3- und 4-Carboxyphenyl; 3- und 4-Carboxamidophenyl, 3-und 4-N-Methylcarboxamidophenyl und 3- und 4-N-Ethylcarboxamidophenyl; 3- und 4-Acetylaminophenyl, 3- und 4-Propionylaminophenyl und 3- und 4-Butyrylaminophenyl; 3- und 4-N-Phenylaminophenyl, 3-und 4-N-(o-Tolyl)aminophenyl, 3- und 4-N-(m-Tolyl)aminophenyl und 3- und 4-N-(p-Tolyl)aminophenyl; 3- und 4-(2-Pyridyl)aminophenyl, 3- und 4-(3-Pyridyl)aminophenyl, 3- und 4-(4-Pyridyl)aminophenyl, 3- und 4-(2-Pyrimidyl)aminophenyl und 4-(4-Pyrimidyl)aminophenyl;
4-Phenylazophenyl, 4-(1-Naphthylazo)phenyl, 4-(2-Naphthylazo)phenyl, 4-(4-Naphthylazo)plienyl, 4-(2-Pyridylazo)phenyl, 4-(3-Pyridylazo)phenyl, 4-(4-Pyridylazo)phenyl, 4-(2-Pyrimidylazo)phenyl, 4-(4-Pyrimidylazo)phenyl und 4-(5-Pyrimidylazo)-phenyl;
Cyclopentyl, 2- und 3-Methylcyclopentyl, 2- und 3-Ethylcyclopentyl, Cyclohexyl, 2-, 3- und 4-Methylcyclohexyl, 2-, 3- und . 4-Ethylcyclohexyl, 3- und 4-Propylcyclohexyl, 3- und 4-Isopropylcyclohexyl, 3- und 4-Butylcyclohexyl, 3- und 4-sec.-Butylcyclohexyl, 3- und 4-tert.-Butylcyclohexyl, Cycloheptyl, 2-, 3- und 4-Methylcycloheptyl, 2-, 3- und 4-Ethylcycloheptyl, 3- und 4-Propylcycloheptyl, 3- und 4-Isopropylcycloheptyl, 3- und 4-Butylcycloheptyl, 3- und 4-sec.-Butylcycloheptyl, 3- und 4-tert.-Butylcycloheptyl, Cyclooctyl, 2-, 3-, 4- und 5-Methylcyclooctyl, 2-, 3-, 4- und 5-Ethylcyclooctyl, 3-, 4- und 5-Propylcyclooctyl, 2-Dioxanyl, 4-Morpholinyl, 2- und 3-Tetrahydrofuryl, 1-, 2- und 3-Pyrrolidinyl und 1-, 2-, 3- und 4-Piperidyl;
Phenoxy, Phenylthio, 1- und 2-Naphthyloxy, 1- und 2-Naphthylthio, 2-, 3- und 4-Pyridyloxy, 2-, 3- und 4-Pyridylthio, 2-, 4- und 5-Pyrimidyloxy und 2-, 4- und 5-Pyrimidylthio;
Dimethylamino, Diethylamino, Dipropylamino, Diisopropylamino, Dibutylamino, Diisobutylamino, Di-tert.-butylamino, Dipentylamino, Dihexylamino, Diphenylamino, Di-o-tolylamino, Di-m-tolylamino, Di-p-tolylamino und Di(4 -cyanophenyl)amino;
1-Propinyl, 1-Butinyl, 1-Pentinyl, 3-Methyl-1-butinyl, 1-Hexinyl, 3- und 4-Methyl-1-pentinyl, 3,3-Dimethyl-1-butinyl, 1-Heptinyl, 3-, 4- und 5-Methyl-1-hexinyl, 3,3-, 3,4- und 4,4-Dimethyl-1-pentinyl, 3-Ethyl-1-pentinyl, 1-Octinyl, 3-, 4-, 5- und 6-Methyl-1-heptinyl, 3,3-, 3,4-, 3,5-, 4,4- und 4,5-Dimethyl-1-hexinyl, 3-, 4- und 5-Ethyl-1-hexinyl, 3-Ethyl-3-methyl-1-pentinyl, 3-Ethyl-4-methyl-1-pentinyl, 3,3,4- und 3,4,4-Trimethyl-1-pentinyl, 1-Noninyl, 1-Decinyl, 1-Undecinyl und 1-Dodecinyl;
4-Cyano-1-butinyl, 5-Cyano-1-pentinyl, 6-Cyano-1-hexinyl, 7-Cyano-1-heptinyl und 8-Cyano-1-octinyl;
4-Hydroxy-1-butinyl, 5-Hydroxy-1-pentinyl, 6-Hydroxy-1-hexinyl, 7-Hydroxy-1-heptinyl, 8-Hydroxy-1-octinyl, 9-Hydroxy-1-noninyl, 10-Hydroxy-1-decinyl, 11-Hydroxy-1-undecinyl und 12-Hydroxy-1-dodecinyl;
4-Carboxy-1-butinyl, 5-Carboxy-1-pentinyl, 6-Carboxy-1-hexinyl, 7-Carboxy-1-heptinyl, 8-Carboxy-1-octinyl, 4-Methylcarboxy-1-butinyl, 5-Methylcarboxy-1-pentinyl, 6-Methylcarboxy-1-hexinyl, 7-Methylcarboxy-1-heptinyl, 8-Methylcarboxy-1-octinyl, 4-Ethylcarboxy-1-butinyl, 5-Ethylcarboxy-1-pentinyl, 6-Ethylcarboxy-1-hexinyl, 7-Ethylcarboxy-1-heptinyl und 8-Ethylcarboxy-1-octinyl;
1-Propenyl, 1-Butenyl, 1-Pentenyl, 3-Methyl-1-butenyl, 1-Hexenyl, 3- und 4-Methyl-1-pentenyl, 3,3-Dimethyl-1-butenyl, 1-Heptenyl, 3-, 4- und 5-Methyl-1-hexenyl, 3,3-, 3,4- und 4,4-Dimethyl-1-pentenyl, 3-Ethyl-1-pentenyl, 1-Octenyl, 3-, 4-, 5- und 6-Methyl-1-heptenyl, 3,3-, 3,4-, 3,5-, 4,4 und 4,5-Dimethyl-1-hexenyl, 3-, 4- und 5-Ethyl-1-hexenyl, 3-Ethyl-3-methyl-1-pentenyl, 3-Ethyl-4-methyl-1-pentenyl, 3,3,4- und 3,4,4-Trimethyl-1-pentenyl, 1-Nonenyl, 1-Decenyl, 1-Undecenyl und 1-Dodecenyl;
4-Cyano-1-butenyl, 5-Cyano-1-pentenyl, 6-Cyano-1-hexenyl, 7-Cyano-1-heptenyl und 8-Cyano-1-octenyl;
4-Hydroxy-1-butenyl, 5-Hydroxy-1-pentenyl, 6-Hydroxy-1-hexenyl, 7-Hydroxy-1-heptenyl, 8-Hydroxy-1-octenyl, 9-Hydroxy-1-nonenyl, 10-Hydroxy-1-decenyl, 11-Hydroxy-1-undecenyl und 12-Hydroxy-1-dodecenyl;
4-Carboxy-1-butenyl, 5-Carboxy-1-pentenyl, 6-Carboxy-1-hexenyl, 7-Carboxy-1-heptenyl, 8-Carboxy-1-octenyl, 4-Methylcarboxy-1-butenyl, 5-Methylcarboxy-1-pentenyl, 6-Methylcarboxy-1-hexenyl, 7-Methylcarboxy-1-heptenyl, 8-Methylcarboxy-1-octenyl, 4-Ethylcarboxy-1-butenyl, 5-Ethylcarboxy-1-pentenyl, 6-Ethylcarboxy-1-hexenyl, 7-Ethylcarboxy-1-heptenyl und 8-Ethylcarboxy-1-octenyl.

Die Herstellung der erfindungsgemäßen Terrylimide Ia, Ia', Ib und Ib', die in 1,6,9,14-Stellung bromiert sind oder 4 gleiche (Het)Aryloxy- oder (Het)Arylthioreste tragen bzw. in 1,6-Stellung und in 9,14-Stellung jeweils 2 gleiche (Het)Aryloxy- oder (Het)Arylthioreste oder in 1,6-Stellung 2 gleiche (Het)Aryloxy-oder (Het)Arylthioreste tragen und in 9,14-Stellung bromiert sind, kann vorteilhaft nach den beiden erfindungsgemäßen Verfahren, ausgehend von den literaturbekannten nichtkernsubstituierten Terrylimiden II bzw. 1,6-disubstituierten Terrylimiden IV, erfolgen.

Dabei werden die Terrylimide II bzw. IV in Schritt a) zunächst durch Umsetzung mit elementarem Brom in die 1,6,9,14-tetrabromierten Terrylimide der Formel Ia' bzw. die 9,14-dibromierten Terrylimide der Formel Ib' überführt.

Die am Terrylenkern durch gegebenenfalls weiter funktionalisierte (Het)Aryloxy- oder (Het)Arylthioreste substituierten Terrylimide Ia bzw. Ib können in einem weiteren Schritt b) durch Umsetzung der bromierten Terrylimide Ia' bzw. Ib' mit einem aromatischen oder heteroaromatischen Alkohol oder Thioalkohol III unter Bromaustausch hergestellt werden.

Schritt a) der erfindungsgemäßen Herstellungsverfahren, die Umsetzung der Terrylimide II bzw. IV mit elementarem Brom, wird in Gegenwart eines Halogenkohlenwasserstoffs als Lösungsmittel vorgenommen.

Dabei sind sowohl aliphatische als auch aromatische halogenierte Kohlenwasserstoffe als Lösungsmittel geeignet, bevorzugt sind die chlorierten Kohlenwasserstoffe. Als Beispiele seien im einzelnen Methylenchlorid, Chloroform, 1,1,2,2-Tetrachlorethan, Chlorbenzol, Dichlorbenzol (alle Isomere) und Trichlorbenzol (alle Isomere) genannt, wobei Chloroform, 1,1,2,2-Tetrachlorethan und Chlorbenzol bevorzugt sind.

Üblicherweise kommen 15 bis 150 g, vorzugsweise 70 bis 100 g, Lösungsmittel je g zu bromierendem Terrylimid II bzw. IV zum Einsatz.

Das Molverhältnis Brom zu Terrylimid hängt von dem eingesetzten Ausgangsmaterial ab. Im allgemeinen werden je einzuführendem Bromatom 1 bis 2 mol, vorzugsweise 1,1 bis 1,5 mol, Brom je mol II bzw. IV eingesetzt.

In der Regel ist die Anwesenheit eines Halogenierungskatalysators nicht erforderlich. Will man jedoch die Bromierungsreaktion beschleunigen (etwa um den Faktor 1,5 bis 3), so empfiehlt es sich, elementares Iod, bevorzugt in einer Menge von 1 bis 5 mol-%, bezogen auf das Terrylimid II bzw. IV, zuzusetzen.

Die Reaktionstemperatur hängt von der Stabilität der Substituenten am Imidstickstoff gegenüber den Halogenierungsbedingungen ab und beträgt im allgemeinen 40 bis 100°C, bei inerten Alkyl- bzw. Cycloalkylsubstituenten vorzugsweise 60 bis 100°C und bei gegenüber den Halogenierungsbedingungen nur eingeschränkt stabilen Aryl- bzw. Hetarylsubstituenten vorzugsweise 50 bis 70°C.

In Abhängigkeit von der Reaktivität des zu bromierenden Terrylimids II bzw. IV und der An- oder Abwesenheit von Iod ist die Bromierung üblicherweise in 2 bis 24 h beendet.

Verfahrenstechnisch geht man in Schritt a) zweckmäßigerweise wie folgt vor:

Man legt Lösungsmittel und Terrylimid II bzw. IV vor, setzt gegebenenfalls den Katalysator und anschließend in 5 bis 10 min die gewünschte Brommenge zu, erwärmt die Mischung unter Rühren auf die gewünschte Reaktionstemperatur und rührt unter Lichtausschluß 2 bis 24 h bei Reaktionstemperatur nach. Nach Entfernen von überschüssigem Brom mit einem kräftigen Stickstoffstrom wird das Lösungsmittel im Vakuum abgezogen. Der Rückstand wird in dem etwa 20-fachen Volumen eines aliphatischen Alkohols, wie Methanol, aufgeschlämmt und über Nacht gerührt. Das ausgefallene Produkt wird dann abfiltriert, vorzugsweise mit dem gleichen Alkohol und anschließend mit Wasser gewaschen und unter Vakuum bei etwa 120°C getrocknet.

Im allgemeinen reicht die Reinheit der so hergestellten 1, 6, 9, 14-tetrabromierten Terrylimide der Formel Ia' bzw. der 9,14-dibromierten Terrylimide der Formel Ib' für die Weiterverarbeitung aus. Gewünschtenfalls kann eine Aufreinigung durch Säulenfiltration an Kieselgel mit Methylenchlorid als Eluens oder durch Extraktion mit einem Lösungsmittel wie Methanol durchgeführt werden.

Terrylimide Ia bzw. Ib, die am Terrylenkern durch gegebenenfalls weiter funktionalisierte (Het)Aryloxy- oder (Het)Arylthioreste substituiert sind, können gemäß Schritt b) der erfindungsgemäßen Verfahren durch Umsetzung der bromierten Terrylimide Ia' bzw. Ib' mit einem aromatischen oder heteroaromatischen Alkohol oder Thioalkohol III in Gegenwart eines inerten stickstoffbasischen Lösungsmittels und einer Base hergestellt werden.

Als inertes stickstoffbasisches Lösungsmittel eignen sich hierfür insbesondere polare Lösungsmittel, vor allem Stickstoffheterocyclen, wie Pyridin, Pyrimidin, Chinolin, Isochinolin, Chinaldin und N-Methylpyrrolidon, sowie Carbonsäureamide, wie N,N-Dimethylformamid und N,N-Dimethylacetamid, wobei N-Methylpyrrolidon bevorzugt ist.

Die Lösungsmittelmenge beträgt abhängig von der Löslichkeit des bromierten Terrylimids Ia' bzw. Ib' üblicherweise 5 bis 100 g, vorzugsweise 10 bis 40 g, je g des Terrylimids Ia' bzw. Ib'.

Als Base sind insbesondere nicht oder nur schwach nukleophile Verbindungen geeignet. Beispiele für solche Basen sind anorganische Basen, z.B. Alkalimetallhydroxide, wie Kalium- und Natriumhydroxid, und Alkalimetallcarbonate, wie Kalium- und Natriumcarbonat, sowie organische Basen, z.B. Alkalimetallalkoholate insbesondere tertiärer Alkohole, wie Lithium-, Natrium- und Kalium-tert.-butanolat, die in wasserfreier Form zum Einsatz kommen.

In der Regel werden 0,8 bis 1,5, bevorzugt 1 bis 1,3, Moläquivalente Base je mol zu substituierendes Bromatom eingesetzt.

Das Molverhältnis bromiertes Terrylimid Ia' bzw. Ib' zu Alkohol bzw. Thioalkohol III hängt ebenfalls von der Anzahl der zu substituierenden Bromatome ab. Im allgemeinen verwendet man 1 bis 2, vorzugsweise 1 bis 1,5, mol III je mol auszutauschendes Bromatom.

Die Reaktionstemperatur liegt üblicherweise im Bereich von 50 bis 200°C, bevorzugt bei 60 bis 140°C.

Es empfiehlt sich, die Umsetzung unter Schutzgas durchzuführen.

Die Reaktionszeit beträgt in Abhängigkeit von der Reaktivität des bromierten Terrylimids Ia' bzw. Ib' etwa 2 bis 48 h.

Verfahrenstechnisch geht man in Schritt b) zweckmäßigerweise so vor, daß man das Lösungsmittel vorlegt, bromiertes Terrylimid Ia' bzw. Ib', Alkohol oder Thioalkohol III und Base zugibt und die erhaltene Lösung bzw. Suspension unter Rühren unter Schutzgas 2 bis 48 h auf die gewünschte Reaktionstemperatur erhitzt.

Die Isolierung des Reaktionsprodukts kann nach Abkühlen auf Raumtemperatur durch direktes Abfiltrieren des bereits ausgefallenen Reaktionsprodukts oder durch Abfiltrieren nach Verdünnen mit dem drei- bis vierfachen Volumen an Wasser, einer verdünnten anorganischen Säure, z.B. 5 bis 10 gew.-%iger Salzsäure, oder eines aliphatischen Alkohols, z.B. Methanol, Waschen zunächst mit wenig Lösungsmittel und anschließend mit Wasser bis zum neutralen Ablauf und Trocknen im Vakuum erfolgen.

Die Herstellung der erfindungsgemäßen Terrylimide Ic, die in 1,6,9,14-Stellung 4 gleiche gewünschtenfalls substituierte Alkyl-, Alkenyl- oder Alkinylreste X³ tragen, kann vorteilhaft nach dem erfindungsgemäßen Verfahren, ausgehend von den ebenfalls erfindungsgemäßen 1,6,9,14-Tetrabromterrylimiden der Formel Ia', erfolgen.

Dabei werden die Tetrabromterrylimide Ia' mit einem Alkin V umgesetzt. Die im Substituent X³ enthaltene ungesättigte Bindung kann gewünschtenfalls zusätzlich reduziert werden.

Die Umsetzung mit dem Alkin V wird in Gegenwart eines aprotischen Lösungsmittels, eines Palladiumkomplexes als Katalysator, eines Kupfersalzes als Cokatalysator und einer Base vorgenommen.

Als Lösungsmittel eignen sich dabei lineare und cyclische aliphatische Ether mit bis zu 10 C-Atomen, wie Diethylether, Din-propylether, Di-n-butylether, 1,2-Dimethoxyethan, 1,2-Diethoxyethan, Dioxan und insbesondere Tetrahydrofuran.

Üblicherweise kommen 30 bis 150 g, vorzugsweise 50 bis 80 g, Lösungsmittel je g Tetrabromterrylimid Ia' zum Einsatz.

Die zugesetzte Base dient gleichzeitig als Cosolvens. Geeignet sind hierfür vor allem mit den Ethern mischbare, organische Stickstoffbasen mit einem Schmelzpunkt unterhalb Raumtemperatur und einem Siedepunkt oberhalb der Reaktionstemperatur.

Bevorzugte Basen sind aliphatische Amine mit bis zu 15 C-Atomen, insbesondere tertiäre Amine, wie Triethylamin, Tri-n-propylamin und Tri-n-butylamin, und cycloaliphatische Amine, wie insbesondere Piperidin.

Üblicherweise werden 0,2 bis 1,5 g, bevorzugt 0,7 bis 1,2 g, Base je g Lösungsmittel zugesetzt.

Als Katalysator finden Palladiumkomplexe Verwendung, die in Verbindung mit Kupfer(I)salzen als Cokatalysator eingesetzt werden.

Beispiele für geeignete Palladiumkomplexe sind Tetrakis(tris-o-tolylphosphin)palladium(0), [1,2-Bis(diphenylphosphino)ethan]-palladium(II)chlorid,[1,1'-Bis(diphenylphosphino)ferrocen]-palladium(II)chlorid, Bis(triethylphosphin)palladium(II)chlorid, Bis(tricyclohexylphosphin)palladium(II)chlorid, Bis(triphenylphosphin)palladium(II)acetat, (2,2'-Bipyridyl)palladium(II)chlorid und insbesondere Tetrakis(triphenylphosphin)palladium(0), Bis(triphenylphosphin)palladium(II)chlorid,Bis(acetonitril) palladium(II)chlorid und Bis(benzonitril)palladium(II)chlorid.

Beispiele für besonders geeignete Kupfer(I)salze sind Kupfer (I) iodid und Kupf er (I) bromid.

In der Regel werden 4 bis 30 mol-%, vorzugsweise 10 bis 20 mol-%, Palladiumkomplex und im allgemeinen 4 bis 40 mol-%, bevorzugt 15 bis 25 mol-%, Kupfer(I)salz, jeweils bezogen auf das Tetrabromterrylimid Ia', eingesetzt.

Das Molverhältnis Tetrabromterrylimid Ia' zu Alkin V liegt in der Regel bei 1 : 4 bis 1 : 8, insbesondere bei 1 : 4 bis 1 : 6.

Die Reaktionstemperatur beträgt üblicherweise 20 bis 140°C, vor allem 40 bis 90°C.

Je nach dem eingesetzten Alkin V kann die Umsetzung bei Normaldruck oder bei einem Überdruck von in der Regel bis zu 50 bar durchgeführt werden. Die Arbeitsweise unter Druck ist erforderlich beim Einsatz flüchtiger Alkine wie Acetylen.

Die Umsetzung dauert im allgemeinen etwa 1 bis 15 h, insbesondere 2 bis 10 h.

In Abhängigkeit von den gewählten Reaktionsbedingungen können Terrylimide Ic hergestellt werden, die als Brückenglied L in den Substituenten X³ 1,2-Ethinylen- oder 1,2-Ethenylenreste enthalten.

Zur Herstellung ungesättigte Bindungen enthaltende Substituenten X³ aufweisender Terrylimide Ic empfiehlt es sich, unter Schutzgas (z.B. Argon oder Stickstoff) zu arbeiten. Beträgt die Reaktionszeit über 4 h und/oder liegt die Reaktionstemperatur über 100°C, so wird die acetylenische Bindung direkt zu ethylenischen Bindung reduziert.

Ethylenreste L enthaltende Terrylimide Ic können durch Nachrühren des Reaktionsgemischs in einer Wasserstoffatmosphäre erhalten werden. Man kann jedoch auch anschließend eine Reduktion der ungesättigten Bindung z.B. mit Wasserstoff unter Palladium/Aktivkohle-Katalyse vornehmen, wobei wie für derartige Reduktionen üblich vorgegangen werden kann (vgl. Larock, Comprehensive Organic Transformations, VCH Publishers New York, 1989, S. 6-17; March, Advanced Organic Chemistry, John Wiley and Sons New York, 4th Edition 1992, S. 775-777; J. Org. Chem. 45, S. 4926-4931 (1980)).

Die Herstellung der erfindungsgemäßen Terrylimide Id, die in 1,6-Stellung durch 2 gleiche gegebenenfalls weiter funktionalisierte (Het)Aryloxy- oder (Het)Arylthioreste substituiert sind und in 9,14-Stellung 2 gleiche gewünschtenfalls substituierte Alkyl-, Alkenyl- oder Alkinylreste X³ tragen, kann vorteilhaft nach dem ebenfalls erfindungsgemäßen Verfahren, ausgehend vom 9,14-dibromierten Terrylimid Ib', analog zu der Herstellung der Terrylimide Ic unter Halbierung der Einsatzmengen an Palladiumkomplex, Kupfer(I)salz und Alkin V erfolgen.

Verfahrenstechnisch geht man bei den Verfahren zur Herstellung der Terrylimide Ic bzw. Id zweckmäßigerweise wie folgt vor:

Man legt eine gerührte Lösung bzw. Suspension des Tetrabromterrylimids Ia bzw. des Dibromterrylimids Ib' in einer Mischung aus Lösungsmittel und Base (beide möglichst wasserfrei) vor, sättigt die Suspension mit Stickstoff durch mehrfaches Entgasen und Belüften mit trockenem Stickstoff, trägt im Stickstoff-Gegenstrom das Kupfer(I)salz, den Palladiumkomplex und das Alkin V ein (flüchtige Alkine wie Acetylen werden abgewogen in die geschlossene Apparatur eingegast) und erhitzt die Reaktionsmischung für die gewünschte Zeit auf die gewünschte Reaktionstemperatur. Gewünschtenfalls wird nun mit Wasserstoff begast und weitere 4 bis 8 h bei der Reaktionstemperatur gerührt. Anschließend (gegebenenfalls nach vorherigem Entspannen) trägt man die Reaktionsmischung direkt, d.h. ohne vorheriges Abkühlen, unter starkem Rühren in das ca. dreifache Volumen einer Mischung aus etwa gleichen Gewichtsteilen konzentrierter Salzsäure und Eis ein, filtriert das Rohprodukt ab, wäscht mit halbkonzentrierter Salzsäure bis zum farblosen Ablauf und anschließend mit Wasser bis zum neutralen Ablauf und trocknet im Vakuum.

Die Herstellung der erfindungsgemäßen Terrylimide der allgemeinen Formel I mit X und/oder Y = Cyano, -NR²₂ kann über literaturbekannte und z.B. in der EP-A-264 543, der WO-A-01/16109 und der älteren deutschen Patentanmeldung 101 08,601.6 beschriebene Methoden erfolgen.

In der Regel haben die erfindungsgemäß erhaltenen Terrylimide I bereits einen so hohen Wertgehalt (> 95%) , daß auf eine weitere Reinigung verzichtet werden kann. Analysenreine Produkte können durch Umkristallisation aus aromatischen Lösungsmitteln, wie Toluol und Xylol, oder halogenierten Kohlenwasserstoffen, wie Methylenchlorid und Chloroform, oder durch Filtration einer Lösung der Produkte in diesen Lösungsmitteln über Kieselgel und anschließendes Einengen hergestellt werden.

Die erfindungsgemäßen Terrylimide I eignen sich hervorragend zur homogenen Einfärbung von hochmolekularen organischen und anorganischen Materialien, insbesondere z.B. von Kunststoffen, vor allem von thermoplastischen Kunststoffen, Lacken und Druckfarben sowie oxidischen Schichtsystemen.

Außerdem eignen sie sich als Dispergierhilfsmittel, Pigmentadditive für organische Pigmente und Zwischenprodukte für die Herstellung von Fluoreszenzfarbmitteln und Pigmentadditiven, zur Herstellung farbiger oder im nahinfraroten Bereich des elektromagnetischen Spektrums absorbierender und/oder emittierender wäßriger Polymerisatdispersionen und als Photoleiter in der Elektrophotographie, als Emitter in Elektro- und Chemilumineszenzanwendungen, als Aktivkomponenten in der Fluoreszenzkonversion, in Biolumineszenzarrays sowie der Photovoltaik und als Laserfarbstoffe.

Die erfindungsgemäßen Terrylimide I absorbieren üblicherweise bei 670 bis 700 nm und emittieren im allgemeinen bei 710 bis 780 nm, ihre Absorption und Emmission sind also deutlich langwelliger als die der bekannten Terrylimide.

### Beispiele

### a) Herstellung der 1,6,9,14-tetrabromierten Terrylimide der Formel Ia'

### Beispiele 1 bis 10

Eine Mischung aus x g (25 mmol) Terrylimid II, y g Iod als Katalysator, 20 g (125 mmol) Brom und v ml des Lösungsmittels L wurde unter Rühren und Lichtausschluß t h auf T°C erhitzt.

Nach Abkühlen der Reaktionslösung auf Raumtemperatur und Ausblasen von überschüssigem Brom mit Stickstoff wurde das Lösungsmittel im Vakuum abgezogen. Das Rohprodukt wurde in 500 ml Methanol aufgeschlämmt, 12 h bei Raumtemperatur gerührt, abfiltriert, mit Methanol bis zum fast farblosen Ablauf und anschließend mit Wasser gewaschen, getrocknet und über Kieselgel mit Dichlormethan als Eluens chromatographiert.

Weitere Einzelheiten zu diesen Versuchen sowie deren Ergebnisse sind in Tabelle 1 zusammengestellt.

**Tabelle 1**

| Bsp | Terrylimid II | x [g] | y [g] | Lösungsmittel L | v [ml] | t [h] | T [°C] | Ausbeute [g]/[%] | Aussehen | Smp. [°C] |
|---|---|---|---|---|---|---|---|---|---|---|
| 1 | N,N'-Didodecylterrylimid | 21,3 | 0,13 | Chlorbenzol | 2000 | 8 | 80 | 23,6/81 | blauviolett, mikrokristallin | >300 |
| 2 | N,N'-Di(5-nonyl)terrylimid | 19,2 | 0,13 | Chlorbenzol | 1400 | 6 | 80 | 22,5/83 | blauviolett, mikrokristallin | >300 |
| 3 | N,N'-Dicyclohexylterrylimid | 17,0 | 0,13 | Chlorbenzol | 1200 | 4 | 80 | 21,9/88 | blau, mikrokristallin | >300 |
| 4 | N,N'-Bis(2,6-diisopropylphenyl)terrylimid | 20,9 | 0,25 | Chloroform | 1800 | 12 | 65 | 22,5/78 | blau, kristallin | >300 |
| 5 | N,N'-Di(2-pyridyl)terrylimid | 16,7 | 0,25 | 1,1,2,2-Tetrachlorethan | 1650 | 16 | 65 | 18,0/73 | dunkelblau, kristallin | >300 |
| 6 | N-(2,6-Diisopropylphenyl)-N'-methylterrylimid | 17,2 | 0,25 | 1,1,2,2-Tetrachlorethan | 1500 | 12 | 65 | 17,8/71 | dunkelblau, kristallin | >300 |
| 7 | N-(2,6-Diisopropylphenyl)-N'-(5-nonyl)terrylimid | 20,0 | 0,25 | Chloroform | 1500 | 12 | 65 | 20,9/75 | blau, mikrokristallin | >300 |
| 8 | N-Cyclohexyl-N'-(2,6-diisopropylphenyl)terrylimid | 18,9 | 0,25 | Chloroform | 1400 | 12 | 65 | 19,8/74 | blau, mikrokristallin | >300 |
| 9 | N-(2,6-Diisopropylphenyl)-N'-(2-pyridyl)terrylimid | 18,8 | 0,25 | Chloroform | 1800 | 12 | 65 | 20,5/77 | dunkelblau, kristallin | >300 |
| 10 | N-Cyclohexyl-N'-methylterrylimid | 15,3 | 0,13 | Chlorbenzol | 1500 | 6 | 80 | 18,5/80 | blau, mikrokristallin | >300 |

Analytische Daten zu Beispiel 4:
Elementaranalyse (Gew.-% ber. /gef .) :
C: 60,55/60,7; H: 3,7/3,7; N: 2,45/2,45; O: 5,55/5,6;
Br: 27,75/27,55;
Masse (FD, 8kV): m/z = 1145,3 [M⁺, 100%];
IR (KBr): ν = 1703 (s, C=O), 1660 (s, C=O) cm⁻¹;
UV/VIS (CHCl₃): λₘₐₓ (ε) = 559 (15850), 605 (46770),
656 (93330) nm.
Analytische Daten zu Beispiel 8:
Elementaranalyse (Gew.-% ber./gef.):
C: 58,25/58,35; H: 3,4/3,4; N: 2,6/2,6; O: 5,95/6,0;
Br: 29,8/29,65;
Masse (FD, 8kV): m/z = 1073,0 [M⁺, 100%];
IR (KBr): ν = 1705 (s, C=O) 1662 (s, C=O) cm⁻¹;
UV/VIS (CHCl₃) : λₘₐₓ (ε) = 556 (16790), 600 (48290),
652 (90070) nm.

### b) Herstellung der 9,14-dibromierten Terrylimide der Formel Ib'

### Beispiele 11 bis 20

Eine Mischung aus x g (25 mmol) Terrylimid IV, y g Iod als Katalysator, 10 g (62,5 mmol) Brom und v ml des Lösungsmittels L wurde unter Rühren und Lichtausschluß t h auf T°C erhitzt. Nach Abkühlen der Reaktionslösung auf Raumtemperatur und Ausblasen von überschüssigem Brom mit Stickstoff wurde das Lösungsmittel im Vakuum abgezogen. Das Rohprodukt wurde in 500 ml Methanol aufgeschlämmt, 12 h bei Raumtemperatur gerührt, abfiltriert, mit Methanol bis zum fast farblosen Ablauf und anschließend mit Wasser gewaschen, getrocknet und über Kieselgel mit Dichlormethan als Eluens chromatographiert.

Weitere Einzelheiten zu diesen Versuchen sowie deren Ergebnisse sind in Tabelle 2 zusammengestellt.

**Tabelle 2**

| Bsp | Terrylimid IV | x [g] | y [g] | Lösungsmittel L | v [ml] | t [h] | T [°C] | Ausbeute [g]/[%] | Aussehen | Smp. [°C] |
|---|---|---|---|---|---|---|---|---|---|---|
| 11 | N,N'-Didodecyl-1,6-diphenoxyterrylimid | 25,9 | 0,13 | 1,1,2,2-Tetrachlorethan | 2500 | 12 | 65 | 22,1/74 | dunkelblau, kristallin | >300 |
| 12 | N,N'-Di(5'-nonyl)-1,6-di(p-tert.-butylphenoxy)terrylimid | 26,6 | 0,13 | Chloroform | 2100 | 12 | 60 | 25,9/85 | schwarzblau, amorph | 295 |
| 13 | N,N'-Dicyclohexyl-1,6-diphenoxyterrylimid | 21,6 | 0,13 | Chloroform | 1600 | 12 | 60 | 20,7/81 | dunkelblau, amorph | >300 |
| 14 | N,N'-Bis(2',6'-diisopropylphenyl)-1,6-diphenoxyterrylimid | 25,5 | 0,13 | Chloroform | 1800 | 12 | 60 | 22,7/77 | dunkelblau, mikrokristallin | >300 |
| 15 | N,N'-Bis(2',6'-diisopropylphenyl)-1,6-di(p-tert.-butylphenoxy)terrylimid | 28,3 | 0,13 | Chloroform | 2000 | 12 | 60 | 25,5/79 | dunkelblau, amorph | 288 |
| 16 | N,N'-Di(2'-pyridyl)-1,6-diphenoxyterrylimid | 21,3 | 0,13 | 1,1,2,2-Tetrachlorethan | 2100 | 12 | 65 | 14,0/72 | schwarzblau, kristallin | >300 |
| 17 | N-(2',6'-Diisopropylphenyl)-N'-methyl-1,6-diphenoxyterrylimid | 21,8 | 0,13 | Chloroform | 1750 | 12 | 65 | 19,8/77 | schwarzblau, mikrokristallin | >300 |
| 18 | N-(2',6'-Diisopropylphenyl)-N'-(5'-nonyl)-1,6-diphenoxyterrylimid | 24,6 | 0,13 | Chloroform | 2000 | 12 | 60 | 21,7/76 | dunkelblau, mikrokristallin | >300 |
| 19 | N-(2',6'-Diisopropylphenyl)-N'-cyclohexyl-1,6-diphenoxyterrylimid | 23,5 | 0,13 | Chloroform | 1750 | 12 | 60 | 22,5/82 | schwarzblau, kristallin | >300 |
| 20 | N-(2',6'-Diisopropylphenyl)-N'-cyclohexyl-1,6-di(p-tert.-butylphenoxy)terrylimid | 26,3 | 0,13 | Chloroform | 1850 | 12 | 60 | 25,4/84 | dunkelblau, mikrokristallin | 256 |

Analytische Daten zu Beispiel 14:
Elementaranalyse (Gew.-% ber./gef.):
C: 71,45/71,6; H: 4,45/4,5; N: 2,4/2,4; O: 8,15/8,2;
Br: 13,55/13,3;
Masse (FD, 8kV): m/z = 1175,7 [M⁺, 100%];
IR (KBr): ν = 1705 (s, C=O), 1662 (s, C=O) cm⁻¹;
UV/VIS (CHCl₃) : λₘₐₓ (ε) = 553 (9750), 616 (51330),
661 (111530) nm.
Analytische Daten zu Beispiel 15:
Elementaranalyse (Gew.-% ber./gef.):
C: 72,65/72,7; H: 5;3/5,3; N: 2,2/2,2; O: 7,45/7,5;
Br: 12,4/12,3;
Masse (FD, 8kV): m/z = 1287,8 [M⁺, 100%];
IR (KBr): ν = 1704 (s, C=O), 1662 (s, C=O) cm⁻¹;
UV/VIS (CHCl₃) : λₘₐₓ (ε) = 551 (10080), 615 (55000),
659 (106780) nm.
Analytische Daten zu Beispiel 19:
Elementaranalyse (Gew.-% ber./gef.):
C: 69,95/70,0; H: 4,2/4,2; N: 2,55/2,6; O: 8,75/8,8;
Br: 14,55/14,4;
Masse (FD, 8kV): m/z = 1198,0 [M⁺, 100%];
IR (KBr): ν = 1705 (s, C=O) , 1660 (s, C=O) cm⁻¹;
UV/VIS (CHCl₃) : λₘₐₓ (ε) = 552 (11040), 616 (53790),
660 (113460) nm.
Analytische Daten zu Beispiel 20:
Elementaranalyse (Gew.-% ber./gef.):
C: 71,4/71,6; H: 5,15/5,2; N: 2,3/2,3; O: 7,95/8,0;
Br: 13,2/12,9;
Masse (FD, 8kV): m/z = 1209,9 [M⁺, 100%];
IR (KBr): ν = 1705 (s, C=O) , 1662 (s, C=O) cm⁻¹;
UV/VIS (CHCl₃) : λₘₐₓ (ε) = 550 (10730), 614 (58490) ,
657 (111110) nm.

### c) Herstellung der 1,6,9,14-tetrasubstituierten Terrylimide der Formel I mit X,Y ≠ Br

### Beispiele 21 bis 50

Eine Mischung aus x g (10 mmol) der tetra- bzw. dibromierten Terrylimide Ia' bzw. Ib' aus Beispiel B, y g (y₁ mmol) Alkohol oder Thioalkohol III, z g (z₁ mmol) wasserfreies Kaliumcarbonat und v ml N-Methylpyrrolidon wurde unter Rühren unter Schutzgasatmosphäre t h auf T °C erhitzt. Nach Abkühlen auf Raumtemperatur wurde das Reaktionsprodukt entweder direkt (Beispiele 22, 30, 32) oder nach Verdünnen mit dem 3-fachen Volumen an einer 5 gew.-%igen Salzsäure (Beispiele 24, 26, 28, 29, 35, 36, 43, 45, 48, 49) oder Methanol (Beispiele 21, 23, 25, 27, 31, 33, 34, 37-42, 44, 46, 47, 50) abfiltriert, zunächst mit wenig Lösungsmittel (Beispiele 22, 30, 32) oder demselben Gemisch aus Lösungs- und Verdünnungsmittel (Beispiele 21, 23-29, 31, 33-50) und anschließend mit Wasser bis zum neutralen Ablauf gewaschen und im Vakuum bei 120°C getrocknet.

Weitere Einzelheiten zu diesen Versuchen sowie deren Ergebnisse sind in Tabelle 3 zusammengestellt.

**Tabelle 3**

| Bsp | x [g] | Terrylimid aus Bsp. | y [g] | y₁ [mmol] | (Thio)Alkohol III | z [g] | z₁ [mmol] | v [ml] | t [h] | T [°C] | Ausbeute [g]/[%] | Aussehen | Smp. [°C] |
|---|---|---|---|---|---|---|---|---|---|---|---|---|---|
| 21 | 11,7 | 1 | 10,3 | 50 | tert.-Octylphenol | 3,45 | 25 | 300 | 8 | 80 | 13,8/83 | dunkelblau, mikrokristallin | >300 |
| 22 | 11,7 | 1 | 6,6 6 | 60 | Thiophenol | 4,15 | 30 | 300 | 12 | 70 | 10,0/78 | schwarzblau, mikrokristallin | >300 |
| 23 | 10,8 | 2 | 4,25 | 45 | Phenol | 3,10 | 22,5 | 250 | 6 | 95 | 9,3/82 | dunkelblau, amorph | >300 |
| 24 | 9,95 | 3 | 7,5 | 50 | tert.-Butylphenol | 3,45 | 25 | 150 | 6 | 95 | 10,8/85 | dunkelblau, amorph | >300 |
| 25 | 9,95 | 3 | 6,6 | 60 | Thiophenol | 4,15 | 30 | 150 | 12 | 70 | 8,8/79 | schwarzblau, amorph | >300 |
| 26 | 9,95 | 3 | 11,0 | 50 | p-Iodphenol | 3,45 | 25 | 150 | 8 | 80 | 12,6/81 | dunkelblau, amorph | >300 |
| 27 | 11,5 | 4 | 4,7 | 50 | Phenol | 3,45 | 25 | 250 | 6 | 95 | 10,5/87 | dunkelblau, kristallin | >300 |
| 28 | 11,5 | 4 | 9,9 | 45 | p-Iodphenol | 3,10 | 22,5 | 250 | 8 | 80 | 13,6/80 | dunkelblau, kristallin | >300 |
| 29 | 11,5 | 4 | 10,3 | 50 | tert.-Octylphenol | 3,45 | 25 | 250 | 8 | 80 | 13,2/80 | dunkelblau, kristallin | >300 |
| 30 | 11,5 | 4 | 6,6 | 60 | Thiophenol | 4,15 | 30 | 250 | 12 | 70 | 9,8/77 | schwarzblau, mikrokristallin | >300 |
| 31 | 9, 85 | 5 | 10,3 | 50 | tert.-Octylphenol | 3,45 | 25 | 350 | 8 | 80 | 12,3/83 | dunkelblau, kristallin | >300 |
| 32 | 9, 85 | 5 | 6,6 | 60 | Thiophenol | 4,15 | 30 | 350 | 12 | 70 | 8,6/78 | schwarzblau, mikrokristallin | >300 |
| 33 | 10,0 | 6 | 7,5 | 50 | tert.-Butylphenol | 3,45 | 25 | 300 | 6 | 95 | 10,8/84 | dunkelblau, kristallin | >300 |
| 34 | 11,2 | 7 | 7,5 | 50 | tert.-Butylphenol | 3,45 | 25 | 200 | 6 | 95 | 11,4/82 | dunkelblau, kristallin | >300 |
| 35 | 10,7 | 8 | 9, 9 | 45 | p-Iodphenol | 3,10 | 22,5 | 250 | 8 | 80 | 13,0/80 | dunkelblau, mikrokristallin | >300 |
| 36 | 10,7 | 8 | 10,3 | 50 | tert.-Octylphenol | 3,45 | 25 | 250 | 8. | 80 | 12,9/82 | dunkelblau, kristallin | >300 |
| 37 | 10,7 | 8 | 6, 6 | 60 | Thiophenol | 4,15 | 30 | 250 | 12 | 70 | 9,0/76 | schwarzblau, mikrokristallin | >300 |
| 38 | 10,7 | 9 | 10,3 | 50 | tert.-Octylphenol | 3,45 | 25 | 300 | 8 | 80 | 12,7/81 | dunkelblau, mikrokristallin | >300 |
| 39 | 10,7 | 9 | 6,6 | 60 | Thiophenol | 4,15 | 30 | 300 | 12 | 70 | 8,9/75 | schwarzblau, mikrokristallin | >300 |
| 40 | 9,3 | 10 | 7,5 | 50 | tert.-Butylphenol | 3,45 | 25 | 350 | 6 | 95 | 9, 6/80 | dunkelblau, mikrokristallin | >300 |
| 41 | 11,95 | 11 | 3,3 | 30 | Thiophenol | 2,10 | 15 | 200 | 12 | 70 | 9,6/77 | schwarzblau, mikrokristallin | >300 |
| 42 | 12,2 | 12 | 3,3 | 30 | Thiophenol | 2,10 | 15 | 150 | 12 | 70 | 9,6/75 | schwarzblau, amorph | >300 |
| 43 | 8,65 | 13 | 5,5 | 25 | p-Iodphenol | 1,75 | 12,5 | 100 | 8 | 80 | 10,5/81 | dunkelblau, amorph | >300 |
| 44 | 10,2 | 14 | 3,3 | 30 | Thiophenol | 2,10 | 15 | 200 | 12 | 70 | 9,8/79 | dunkelblau, mikrokristallin | >300 |
| 45 | 11,3 | 15 | 5,5 | 25 | p-Iodphenol | 1, 75 . | 12,5 | 150 | 8 | 80 | 13,0/83 | dunkelblau, amorph | >300 |
| 46 | 8,55 | 16 | 3,3 | 30 | Thiophenol | 2,10 | 15 | 200 | 12 | 70 | 8,1/76 | dunkelblau, mikrokristallin | >300 |
| 47 | 10,3 | 17 | 3,3 | 30 | Thiophenol | 2,10 | 15 | 200 | 12 | 70 | 8,6/79 | schwarzblau, mikrokristallin | >300 |
| 48 | 11,45 | 18 | 5,5 | 25 | p-Iodphenol | 1,75 | 12,5 | 200 | 8 | 80 | 11,7/82 | dunkelblau, amorph | >300 |
| 49 | 9,4 | 19 | 5,5 | 25 | p-Iodphenol | 1,75 | 12,5 | 150 | 8 | 80 | 11,7/85 | dunkelblau, amorph | >300 |
| 50 | 10,55 | 20 | 3,3 | 30 | Thiophenol | 2,10 | 15 | 150 | 12 | 70 | 10,2/80 | schwarzblau, amorph | >300 |

Analytische Daten zu Beispiel 27:
Elementaranalyse (Gew.-% ber./gef.):
C: 81,85/81,8; H: 5,2/5,2; N: 2,3/2,35; O: 10,65/10,65;
Masse (FD, 8kV): m/z = 1202,7 [M⁺, 100%];
IR (KBr): ν = 1709 (s, C=O), 1668 (s, C=O) cm⁻¹;
UV/VIS (CHCl₃) : λₘₐₓ (ε) = 630 (61010), 678 (143210) nm.
Analytische Daten zu Beispiel 28:
Elementaranalyse (Gew.-% ber./gef.): .
C: 57,7/57,6; H: 3,4/3,4; N: 1,65/1,65; O: 7,5/7,55;
I: 29,75/29,8;
Masse (FD, 8kV): m/z = 1706,5 [M⁺, 100%];
IR (KBr): ν = 1708 (s, C=O), 1669 (s, C=O) cm⁻¹;
UV/VIS (CHCl₃): λₘₐₓ (ε) = 626 (50330), 675 (124000) nm.
Analytische Daten zu Beispiel 29:
Elementaranalyse (Gew.-% ber./gef.):
C: 82,85/82,8; H: 7,7/7,7; N: 1,7/1,7; O: 7,75/7,8;
Masse (FD, 8kV): m/z = 1651,2 [M⁺, 100%];
IR (KBr): ν = 1708 (s, C=O), 1668 (s, C=O) cm⁻¹;
UV/VIS (CHCl₃) : λₘₐₓ (ε) = 625 (52930), 677 (128770) nm.
Analytische Daten zu Beispiel 30:
Elementaranalyse (Gew.-% ber./gef.):
C: 77, 7/77,5; H: 4,95/5,0; N: 2,2/2,2; O: 5,05/5,1; S: 10,1/10,2;
Masse (FD, 8kV): m/z = 1266,9 [M⁺, 100%];
IR (KBr): ν = 1706 (s, C=O), 1667 (s, C=O) cm⁻¹;
UV/VIS (CHCl₃) : λₘₐₓ (ε) = 638 (67860), 694 (157030) nm.
Analytische Daten zu Beispiel 35:
Elementaranalyse (Gew.-% ber./gef.):
C: 56,05/55,9; H: 3,2/3,2; N: 1,7/1,7; O: 7,85/7,9; I: 31,2/31,3;
Masse (FD, 8kV): m/z = 1628,4 [M⁺, 100%];
IR (KBr): ν = 1709 (s, C=O), 1667 (s, C=O) cm⁻¹;
UV/VIS (CHCl₃) : λₘₐₓ (ε) = 624 (53220), 667 (129770) nm.
Analytische Daten zu Beispiel 36:
Elementaranalyse (Gew.-% ber./gef.):
C: 82,4/82,3; H: 7,7/7,7; N: 1,8/1,8; O: 8,1/8,2;
Masse (FD, 8kV): m/z = 1573,5 [M⁺, 1.00%];
IR (KBr): ν = 1709 (s, C=O), 1667 (s, C=O) cm⁻¹;
UV/VIS (CHCl₃): λₘₐₓ (ε) = 624 (54010), 673 (129770) nm.
Analytische Daten zu Beispiel 37:
Elementaranalyse (Gew.-% ber./gef.):
C: 76,75/76,6; H: 4,75/4,75; N: 2,35/2,35; O: 5,4/5,45;
S: 10,75/10,85;
Masse (FD, 8kV): m/z = 1188,9 [M⁺, 100%];
IR (KBr): ν = 1707 (s, C=O) , 1668 (s, C=O) cm⁻¹;
UV/VIS (CHCl₃) : λₘₐₓ (ε) = 635 (68880), 691 (160210) nm.
Analytische Daten zu Beispiel 49:
Elementaranalyse (Gew.-% ber./gef.):
C: 66,3/66,2; H: 3,95/3,95; N: 2,05/2,1; O: 9,3/9,35;
I: 18,4/18,4;
Masse (FD, 8kV): m/z = 1376,3 [M⁺, 100%];
IR (KBr): ν = 1709 (s, C=O) , 1667 (s, C=O) cm⁻¹;
UV/VIS (CHCl₃): λₘₐₓ (ε) = 624 (56040), 672 (134830) nm.
Analytische Daten zu Beispiel 50:
Elementaranalyse (Gew.-% ber./gef.) :
C: 79,5/79,3; H: 5,7/5,7; N: 2,2/2,2; O: 7,55 /7,7; S: 5,05/5,1;
Masse (FD, 8kV): m/z = 1269,0 [M⁺, 100%];
IR (KBr): ν = 1708 (s, C=O), 1669 (s, C=O) cm⁻¹;
UV/VIS (CHCl₃) : λₘₐₓ (ε) = 630 (61650), 686 (143410) nm.

## Patentansprüche (Patentansprüche für folgende(n) Vertragsstaat(en): AT, BE, CH, CY, DE, DK, ES, FI, FR, GB, GR, IE, IT, LI, LU, MC, NL, PT, SE, TR)

1. 1,6,9,14-tetrasubstituierte Terrylentetracarbonsäurediimide der allgemeinen Formel I 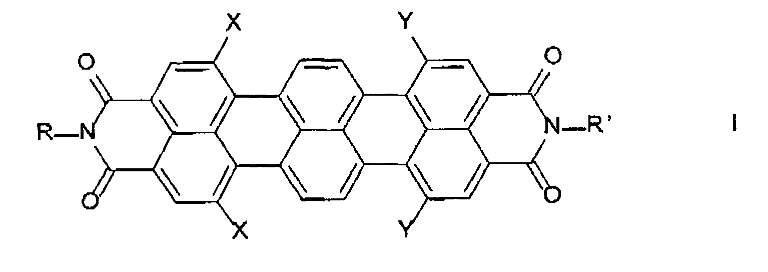 in der die Variablen folgende Bedeutung haben:
X, Y unabhängig voneinander
Brom; Cyano;
Aryloxy, Arylthio, Hetaryloxy oder Hetarylthio, das jeweils ein- oder mehrfach durch C₁-C₁₂-Alkyl, C₁-C₁₂-Alkoxy, -COOR¹, -SO₃R¹, Halogen, Hydroxy, Carboxy, Cyano, -CONHR² und/oder -NHCOR² substituiert sein kann;
einen Rest der Formel -L-R³;
einen Rest der Formel -NR²₂;
R, R' unabhängig voneinander
Wasserstoff;
C₁-C₃₀-Alkyl, dessen Kohlenstoffkette durch eine oder mehrere Gruppierungen -O-, -S-, -NR¹-, -CO- und/oder -SO₂- unterbrochen sein kann und das durch Cyano, C₁-C₆-Alkoxy, Aryl, das durch C₁-C₁₈-Alkyl oder C₁-C₆-Alkoxy substituiert sein kann, und/oder einen über ein Stickstoffatom gebundenen 5- bis 7-gliedrigen heterocyclischen Rest, der weitere Heteroatome enthalten und aromatisch sein kann, ein- oder mehrfach substituiert sein kann;
C₅-C₈-Cycloalkyl, dessen Kohlenstoffgerüst durch eine oder mehrere Gruppierungen -O-, -S- und/oder -NR¹- unterbrochen und/oder das durch C₁-C₆-Alkyl ein- oder mehrfach substituiert sein kann;
Aryl oder Hetaryl, das durch C₁-C₁₈-Alkyl, C₁-C₆-Alkoxy, Cyano, -CONHR², -NHCOR² und/oder Aryl- oder Hetarylazo, das jeweils durch C₁-C₁₀-Alkyl, C₁-C₆-Alkoxy oder Cyano substituiert sein kann, ein- oder mehrfach substituiert sein kann;
L 1,2-Ethylen, 1,2-Ethenylen oder 1,2-Ethinylen;
R¹ Wasserstoff oder C₁-C₆-Alkyl;
R² Wasserstoff; C₁-C₁₈-Alkyl; Aryl oder Hetaryl, das jeweils durch C₁-C₆-Alkyl, C₁-C₆-Alkoxy, Halogen, Hydroxy, Carboxy oder Cyano substituiert sein kann;
R³ C₁-C₁₈-Alkyl, dessen Kohlenstoffkette durch eine oder mehrere Gruppierungen -O-, -S-, -NR¹-, -CO- und/oder -SO₂- unterbrochen sein kann und das durch -COOR¹,-SO₃R¹, Hydroxy, Cyano, C₁-C₆-Alkoxy, Aryl, das durch C₁C₁₈-Alkyl oder C₁-C₆-Alkoxy substituiert sein kann, und/oder einen über ein Stickstoffatom gebundenen 5-bis 7-gliedrigen heterocyclischen Rest, der weitere Heteroatome enthalten und aromatisch sein kann, ein-oder mehrfach substituiert sein kann,
mit der Maßgabe, dass N,N'-Bis(2,6-diisopropylphenyl)-1,6,9,14-tetrabromterrylen-3,4:11,12-tetracarbonsäurediimid, N,N'-Bis(2,6-diisopropylphenyl)-1,6,9,14-tetra(p-terc-octylphenoxy)terrylen-3,4:11,12-tetracarbonsäurediimid, N-Cyclohexyl-N'-(2,6-diisopropylphenyl)-1,6,9,14-tetrabromterrylen-3,4:11,12-tetracarbonsäurediimid, N-Cyclohexyl-N'-(2,6-diisopropylphenyl)-1,6,9,14-tetra(p-tert.-octylphenoxy) terrylen-3, 4:11, 12-tetracarbonsäurediimid ausgeschlossen sind.

2. 1,6,9,14-tetrasubstituierte Terrylentetracarbonsäurediimide der Formel I nach Anspruch 1, in der die Variablen folgende Bedeutung haben:
X, Y unabhängig voneinander
Brom; Cyano;
Phenoxy, Phenylthio, Pyridyloxy oder Pyridylthio, das jeweils ein- oder mehrfach durch C₁-C₁₂-Alkyl, C₁-C₁₂-Alkoxy, -COOR¹, Halogen, Hydroxy, Carboxy, Cyano,-CONHR² und/oder -NHCOR² substituiert sein kann;
einen Rest der Formel -L-R³;
R, R' unabhängig voneinander
Wasserstoff;
C₁-C₃₀-Alkyl, dessen Kohlenstoffkette durch eine oder mehrere Gruppierungen -O- und/oder -CO- unterbrochen sein kann und das durch Cyano, C₁-C₆-Alkoxy und/oder Aryl, das durch C₁-C₁₈-Alkyl oder C₁-C₆-Alkoxy substituiert sein kann, ein- oder mehrfach substituiert sein kann;
C₅-C₈-Cycloalkyl, das durch C₁-C₆-Alkyl ein- oder mehrfach substituiert sein kann;
phenyl, Naphthyl oder Pyridyl, das durch C₁-C₁₆-Alkyl, C₁-C₆-Alkoxy, Cyano, -CONHR² und/oder -NHCOR² ein- oder mehrfach substituiert sein kann;
L 1,2-Ethenylen oder 1,2-Ethinylen;
R¹ Wasserstoff oder C₁-C₆-Alkyl;
R² Wasserstoff; C₁-C₁₈-Alkyl; Phenyl, das jeweils durch C₁-C₆-Alkyl, C₁-C₆-Alkoxy, Halogen, Hydroxy, Carboxy oder Cyano substituiert sein kann;
R³ C₁-C₁₈-Alkyl, dessen Kohlenstoffkette durch eine oder mehrere Gruppierungen -O- und/oder -CO- unterbrochen sein kann und das durch -COOR¹, Hydroxy, Cyano, C₁-C₆-Alkoxy und/oder Aryl, das durch C₁-C₁₈-Alkyl oder C₁-C₆-Alkoxy substituiert sein kann, ein- oder mehrfach substituiert sein kann.
mit der Maßgabe, dass N,N'-Bis(2,6-diisopropylphenyl) - 1,6,9,14-tetrabromterrylen-3,4:11,12-tetracarbonsäurediimid, N,N'-Bis(2,6-diisopropylphenyl)-1,6,9,14-tetra(p-tert.-octylphenoxy)terrylen-3,4:11,12-tetracarbonsäurediimid, N-Cyclohexyl-N'-(2,6-diisopropylphenyl)-1,6,9,14-tetrabromterrylen-3,4:11,12-tetracarbonsäurediimid, N-Cyclohexyl-N'-(2,6-diisopropylphenyl)-1,6,9,14-tetra(p-tert,-octylphenoxy)terrylen-3,4:11,12-tetracarbonsäurediimid ausgeschlossen sind.

3. verfahren zur Herstellung von 1,6,9,14-tetrasubstituierten Terrylentetracarbonsäurediimiden der allgemeinen Formel Ia 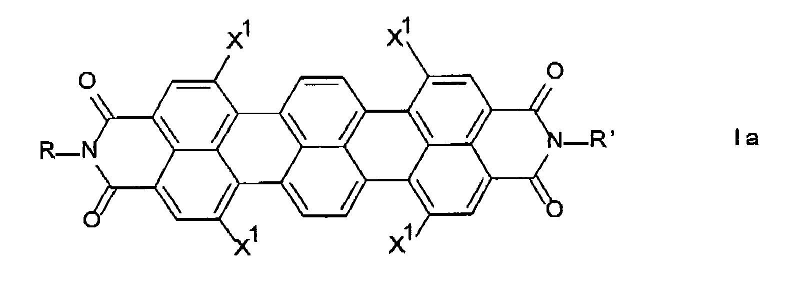 in der die Variablen folgende Bedeutung haben:
X¹ Brom; Aryloxy, Arylthio, Hetaryloxy oder Hetarylthio, das jeweils ein- oder mehrfach durch C₁-C₁₂-Alkyl, C₁-C₁₂-Alkoxy, -COOR², -SO₃R¹, Halogen, Hydroxy, Carboxy, Cyano, -CONHR² und/oder -NHCOR² substituiert sein kann;
R, R' unabhängig voneinander
Wasserstoff;
C₁-C₃₀-Alkyl, dessen Kohlenstoffkette durch eine oder mehrere Gruppierungen -O-, -S-, -NR¹-, -CO- und/oder -SO₂- unterbrochen sein kann und das durch Cyano, C₁-C₆-Alkoxy, Aryl, das durch C₁-C₁₈-Alkyl oder C₁-C₆-Alkoxy substituiert sein kann, und/oder einen über ein stickstoffatom gebundenen 5- bis 7-gliedrigen heterocyclischen Rest, der weitere Heteroatome enthalten und aromatisch sein kann, ein- oder mehrfach substituiert sein kann;
C₅-C₈-Cycloalkyl, dessen Kohlenstoffgerüst durch eine oder mehrere Gruppierungen -O-, -S- und/oder -NR¹-unterbrochen und/oder das durch C₁-C₆-Alkyl ein- oder mehrfach substituiert sein kann;
Aryl oder Hetaryl, das durch C₁-C₁₈-Alkyl, C₁-C₆-Alkoxy, Cyano, -CONHR², -NHCOR² und/oder Aryl- oder Hetarylazo, das jeweils durch C₁-C₁₀-Alkyl, C₁-C₆-Alkoxy oder Cyano substituiert sein kann, ein- oder mehrfach substituiert sein kann;
R¹ Wasserstoff oder C₁-C₆-Alkyl;
R² Wasserstoff; C₁-C₁₈-Alkyl; Aryl oder Hetaryl, das jeweils durch C₁-C₆-Alkyl, C₁-C₆-Alkoxy, Halogen, Hydroxy, Carboxy oder Cyano substituiert sein kann,
mit der Maßgabe, dass N,N'-Bis(2,6-diisopropylphenyl)-1,6,9,14-tetrabromterrylen-3,4:11,12-tetracarbonsäurediimid, N,N'-Bis(2,6-diisopropylphenyl)-1,6,9,14-tetra(p-tert.-octylphenoxy)terrylen-3,4:21,12-tetracarbonsäurediimid, N-Cyclohexyl-N'-(2,6-diisopropylphenyl)-1,6,9,14-tetrabromterrylen-3,4:11,12-tetracarbonsäurediimid, N-Cyclohexyl-N'-(2,6-diisopropylphenyl)-1,6,9,14-tetra(p-tert.-octylphenoxy)terrylen-3,4:11,12-tetracarbonsäurediimid ausgeschlossen sind,
**dadurch gekennzeichnet, daß** man
a) ein Terrylentetracarbonsäurediimid der allgemeinen Formel II 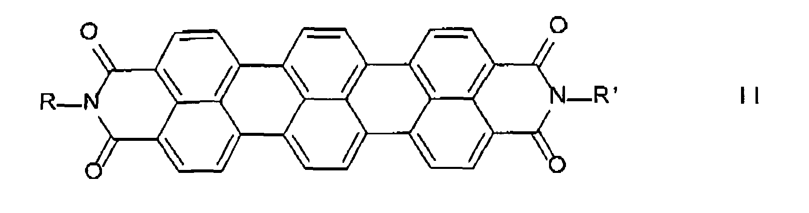 mit elementarem Brom in Gegenwart eines Halogenkohlenwasserstoffs als Lösungsmittel zum 1,6,9,14-Tetrabromterrylentetracarbonsäurediimid der allgemeinen Formel Ia' 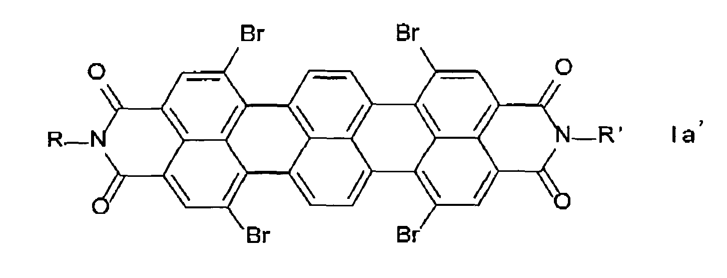 umsetzt und gewünschtenfalls
b) das in Schritt a) erhaltene Tetrabromterrylentetracarbonsäurediimid Ia' in Gegenwart eines inerten stickstoffbasischen Lösungsmittels und einer Base mit einem Alkohol oder Thioalkohol der allgemeinen Formel III mit X¹ ≠ Br 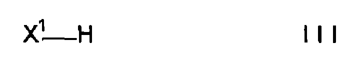 in das 1,6,9,14-tetrasubstituierte Terrylentetracarbonsäurediimid der Formel Ia mit X¹ ≠ Brom überführt, mit der Maßgabe, dass N, N'-Bis(2,6-dii.sopropylphenyl)-1,6,9,4-tetra(p-tert.-octylphenyl)-terrylen-3,4:11,12-dicarbonsäurediimid und N-Cyclohexyl-N'-(2,6-diisopropylphenyl)-1,6,9,14-tetra-(p-tert.octylphenoxy)terrylen-3,4:11,12-dicarbonsäurediimid ausgenommen sind.

4. Verfahren zur Herstellung von 1,6,9,14-tetrasubstituierten Terrylentetracarbonsäurediimiden der allgemeinen Formel Ib 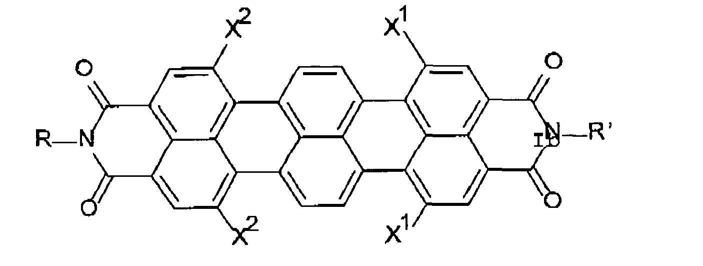 in der die Variablen folgende Bedeutung haben:
X² Aryloxy, Arylthio, Hetaryloxy oder Hetarylthio, das jeweils ein- oder mehrfach durch C₁-C₁₂-Alkyl, C₁-C₁₂-Alkoxy, -COOR¹, -SO₃R¹, Cyano, -CONHR⁴ und/oder -NHCOR⁴ substituiert sein kann;
X¹ Brom; Aryloxy, Arylthio, Hetaryloxy oder Hetarylthio, das jeweils ein- oder mehrfach durch C₁-C₁₂-Alkyl, C₁-C₁₂-Alkoxy, -COOR¹, -SO₃R¹, Halogen, Hydroxy, Carboxy, Cyano, -CONHR² und/oder -NHCOR² substituiert sein kann;
R, R' unabhängig voneinander
wasserstoff;
C₁-C₃₀-Alkyl, dessen Kohlenstoffkette durch eine oder mehrere Gruppierungen -O-, -S-, -NR¹-, -CO- und/oder -SO₂- unterbrochen sein kann und das durch Cyano, C₁-C₆-Alkoxy, Aryl, das durch C₁-C₁₈-Alkyl oder C₁-C₆-Alkoxy substituiert sein kann, und/oder einen über ein Stickstoffatom gebundenen 5- bis 7-gliedrigen heterocyclischen Rest, der weitere Heteroatome enthalten und aromatisch sein kann, ein- oder mehrfach substituiert sein kann;
C₅-C₈-Cycloalkyl, dessen Kohlenstoffgerüst durch eine oder mehrere Gruppierungen -O-, -S- und/oder -NR¹-unterbrochen und/oder das durch C₁-C₆-Alkyl ein- oder mehrfach substituiert sein kann;
Aryl oder Hetaryl, das durch C₁-C₁₈-Alkyl, C₁-C₆-Alkoxy, Cyano, -CONHR², -NHCOR² und/oder Aryl- oder Hetarylazo, das jeweils durch C₁-C₁₀-Alkyl, C₁-C₆-Alkoxy oder Cyano substituiert sein kann, ein- oder mehrfach substituiert sein kann;
R¹ Wasserstoff oder C₁-C₆-Alkyl;
R² wasserstoff; C₁-C₁₈-Alkyl; Aryl oder Hetaryl, das jeweils durch C₁-C₆-Alkyl, C₁-C₆-Alkoxy, Halogen, Hydroxy, Carboxy oder Cyano substituiert sein kann,
R⁴ C₁-C₁₈-Alkyl; Aryl oder Hetaryl, das jeweils durch C₁-C₆-Alkyl, C₁-C₆-Alkoxy, Hydroxy oder Cyano substituiert sein kann,
**dadurch gekennzeichnet, daß** man
a) ein 1,6-disubstiutiertes Terrylentetracarbonsäurediimid der allgemeinen Formel IV 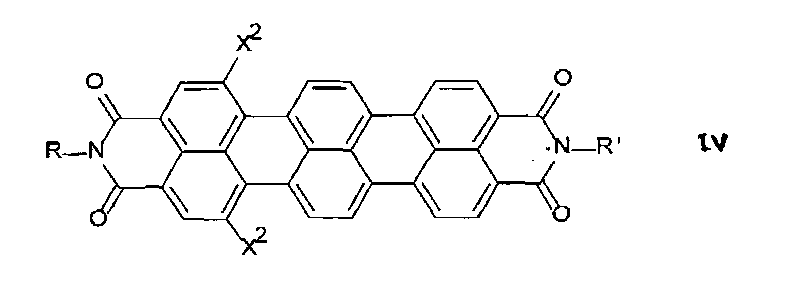 mit elementarem Brom in Gegenwart eines Halogenkohlenwasserstoffs als Lösungsmittel zum 9,14-dibromierten Terrylentetracarbonsäurediimid der allgemeinen Formel Ib' 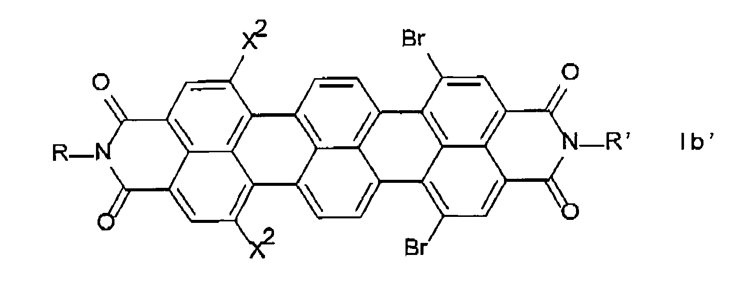 umsetzt und gewünschtenfalls
b) das in Schritt a) erhaltene 9,14-dibromierte Terrylentetracarbonsäurediimid Ib' in Gegenwart eines inerten stickstoffbasischen Lösungsmittels und einer Base mit einem Alkohol oder Thioalkohol der allgemeinen Formel III mit X¹ ≠ Brom 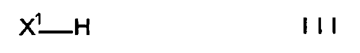 in das 1,6,9,14-tetrasubstituierte Terrylentetracarbonsäurediimid der Formel Ib mit X¹ ≠ Brom überführt.

5. Verfahren zur Herstellung von 1,6,9,14-tetrasubstituierten Terrylentetracarbonsäurediimiden der allgemeinen Formel Ic 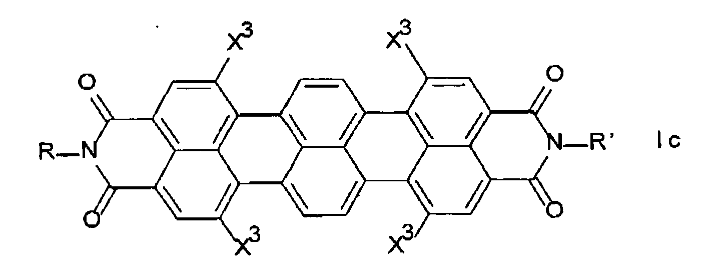 in der die Variablen folgende Bedeutung haben:
x³ einen Rest der Formel -L-R³;
R, R' unabhängig voneinander
Wasserstoff;
C₁-C₃₀-Alkyl, dessen Kohlenstoffkette durch eine oder mehrere Gruppierungen -O-, -S-, -NR¹-, -CO- und/oder -SO₂- unterbrochen sein kann und das durch Cyano, C₁-C₆-Alkoxy, Aryl, das durch C₁-C₁₈-Alkyl oder C₁-C₆-Alkoxy substituiert sein kann, und/oder einen über ein Stickstoffatom gebundenen 5- bis 7-gliedrigen heterocyclischen Rest, der weitere Heteroatome enthalten und aromatisch sein kann, ein- oder mehrfach substituiert sein kann;
C₅-C₈-Cycloalkyl, dessen Kohlenstoffgerüst durch eine oder mehrere Gruppierungen -O-, -S- und/oder -NR¹- unterbrochen und/oder das durch C₁-C₆-Alkyl ein- oder mehrfach substituiert sein kann;
Aryl oder Hetaryl, das durch C₁-C₁₆-Alkyl, C₁-C₆-Alkoxy, Cyano, -CONHR², -NHCOR² und/oder Aryl- oder Hetarylazo, das jeweils durch C₁-C₁₀-Alkyl, C₁-C₆-Alkoxy oder Cyano substituiert sein kann, ein- oder mehrfach substituiert sein kann;
L 1,2-Ethylen, 1, 2-Ethenylen oder 1,2-Ethinylen;
R¹ Wasserstoff oder C₁-C₆-Alkyl;
R² Wasserstoff; C₁-C₁₈-Alkyl; Aryl oder Hetaryl, das jeweils durch C₁-C₆-Alkyl, C₁-C₆-Alkoxy, Halogen, Hydroxy, Carboxy oder Cyano substituiert sein kann;
R³ C₁-C₁₈-Alkyl, dessen Kohlenstoffkette durch eine oder mehrere Gruppierungen -O-, -S-, -NR¹-, -CO- und/oder -SO₂- unterbrochen sein kann und das durch -COOR¹,-SO₃R¹, Hydroxy, Cyano, C₁-C₆-Alkoxy, Aryl, das durch C₁-C₁₆-Alkyl oder C₁-C₆-Alkoxy substituiert sein kann, und/oder einen über ein Stickstoffatom gebundenen 5-bis 7-gliedrigen heterocyclischen Rest, der weitere Heteroatome enthalten und aromatisch sein kann, ein-oder mehrfach substituiert sein kann,
**dadurch gekennzeichnet, daß** man ein 1,6,9,14-Tetrabromterrylentetracarbonsäurediimid der allgemeinen Formel Ia' 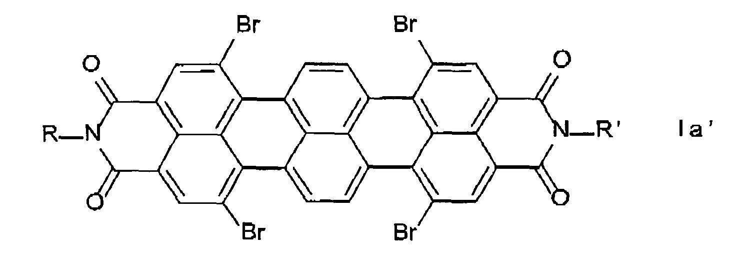 in Gegenwart eines aprotischen Lösungsmittels, eines Palladiumkomplexes als Katalysator, eines Kupfersalzes als Cokatalysator und einer Base mit einem 1-Alkin der allgemeinen Formel V
H-C≡C-R³ V
umsetzt und gewünschtenfalls die in den Resten X³ des erhaltenen 1,6,9,14-tetrasubstituierten Terrylentetracarbonsäurediimids enthaltenen ungesättigten Bindungen zusätzlich reduziert.

6. verfahren zur Herstellung von 1,6,9,14-tetrasubstituierten Terrylentetracarbonsaurediimiden der allgemeinen Formel Id 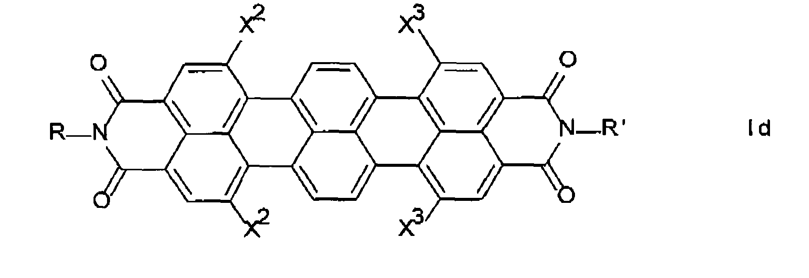 in der die Variablen folgende Bedeutung haben:
x² Aryloxy, Arylthio, Hetaryloxy oder Hetarylthio, das jeweils ein- oder mehrfach durch C₁-C₁₂-Alkyl, C₁-C₁₂-Alkoxy, -COOR¹, -SO₃R¹, Cyano, -CONHR⁴ und/oder -NHCOR⁴ substituiert sein kann;
X³ einen Rest der Formel -L-R³;
R, R' gleich oder verschieden und unabhängig voneinander
Wasserstoff;
C₁-C₃₀-Alkyl, dessen Kohlenstoffkette durch eine oder mehrere Gruppierungen -O-, -S-, -NR¹-, -CO- und/oder -SO₂- unterbrochen sein kann und das durch Cyano, C₁-C₆-Alkoxy, Aryl, das durch C₁-C₁₈-Alkyl oder C₁-C₆-Alkoxy substituiert sein kann, und/oder einen über ein Stickstoffatom gebundenen 5- bis 7-gliedrigen heterocyclischen Rest, der weitere Heteroatome enthalten und aromatisch sein kann, ein- oder mehrfach substituiert sein kann;
C₅-C₈-Cycloakyl, dessen Kohlenstoffgerüst durch eine oder mehrere Gruppierungen -O-, -S- und/oder -NR¹-unterbrochen und/oder das durch C₁-C₆-Alkyl ein- oder mehrfach substituiert sein kann;
Aryl oder Hetaryl, das durch C₁-C₁₈-Alkyl, C₁-C₆-Alkoxy, Cyano, -CONHR², -NHCOR² und/oder Aryl- oder Hetarylazo, das jeweils durch C₁-C₁₀-Alkyl, C₁-C₆-Alkoxy oder Cyano substituiert sein kann, ein- oder mehrfach substituiert sein kann;
L 1,2-Ethylen, 1,2-Ethenylen oder 1,2-Ethinylen;
R¹ Wasserstoff oder C₁-C₆-Alkyl;
R² Wasserstoff; C₁-C₁₈-Alkyl; Aryl oder Hetaryl, das jeweils durch C₁-C₆-Alkyl, C₁-C₆-Alkoxy, Halogen, Hydroxy, Carboxy oder Cyano substituiert sein kann;
R³ C₁-C₁₈-Alkyl, dessen Kohlenstoffkette durch eine oder mehrere Gruppierungen -O-, -S-, -NR¹-, -CO- und/oder -SO₂- unterbrochen sein kann und das durch -COOR¹,-SO₃R¹, Hydroxy, Cyano, C₁-C₆-Alkoxy, Aryl, das durch C₁-C₁₈-Alkyl oder C₁-C₆-Alkoxy substituiert sein kann, und/oder einen über ein Stickstoffatom gebundenen 5-bis 7-gliedrigen heterocyclischen Rest, der weitere Heteroatome enthalten und aromatisch sein kann, ein-oder mehrfach substituiert sein kann;
R⁴ C₁-C₁₈-Alkyl; Aryl oder Hetaryl, das jeweils durch C₁-C₆-Alkyl, C₁-C₆-Alkoxy, Halogen, Hydroxy, Carboxy oder Cyano substituiert sein kann,
**dadurch gekennzeichnet, daß** man ein 9,14-dibromiertes Terrylentetracarbonsäurediimid der allgemeinen Formel Ib' 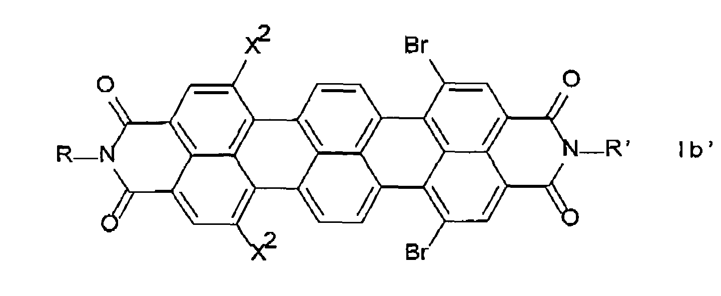 in Gegenwart eines aprotischen Lösungsmittels, eines Palladiumkomplexes als Katalysator, eines Kupfersalzes als Cokatalysator und einer Base mit einem 1-Alkin der allgemeinen Formel V
H-C≡C-R³ V
umsetzt und gewünschtenfalls die in den Resten X³ des erhaltenen 1,6,9,14-tetrasubstituierten Terrylentetracarbonsäurediimids enthaltenen ungesättigten Bindungen zusätzlich reduziert.

7. Verwendung von 1,6,9,14-tetrasubstituierten Terrylentetracarbonsäurediimiden der Formel 1 gemäß Anspruch 1 oder 2 zur Einfärbung von hochmolekularen organischen und anorganischen Materialien.

8. verwendung von 1,6,9,14-tetrasubstituierten Terrylentetracarbonsäurediimiden der Formel 1 gemäß Anspruch 1 oder 2 als Dispergierhilfsmittel, Pigmentadditive für organische Pigmente und Zwischenprodukte für die Herstellung von Fluoreszenzfarbmitteln und Pigmentadditiven.

9. Verwendung von 1,6,9,14-tetrasubstituierten Terrylentetracarbonsäurediimiden der Formel 1 gemäß Anspruch 1 oder 2 zur Herstellung farbiger oder im nahinfraroten Bereich des elektromagnetischen Spektrums absorbierender und/oder emittierender wäßriger Polymerisatdispersionen.

10. Verwendung von 1,6,9,14-tetrasubstituierten Terrylentetracarbonsäurediimiden der Formel 1 gemäß Anspruch 1 oder 2 als Emitter in Elektro- und Chemilumineszenzanwendungen, als Aktivkomponenten in der Fluoreszenzkonversion, in Biolumineszenzarrays und in der Photovoltaik und als Laserfarbstoffe.

## Patentansprüche (Patentansprüche für folgende(n) Vertragsstaat(en): BG, CZ, EE, HU, RO, SI, SK)

1. 1,6,9,14-tetrasubstituierte Terrylentetracarbonsäurediimide der allgemeinen Formel I 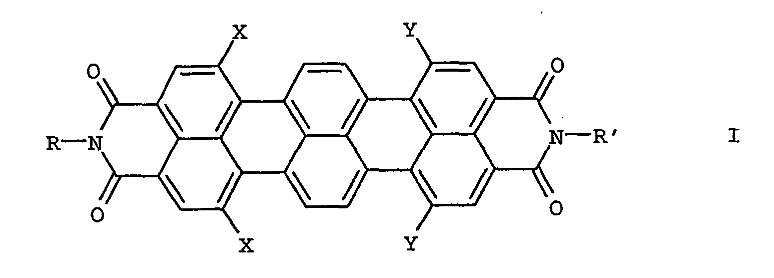 in der die Variablen folgende Bedeutung haben:
X, Y unabhängig voneinander
Brom; Cyano;
Aryloxy, Arylthio, Hetaryloxy oder Hetarylthio, das jeweils ein- oder mehrfach durch C₁-C₁₂-Alkyl, C₁-C₁₂-Alkoxy, -COOR¹, -SO₃R¹, Halogen, Hydroxy, Carboxy, Cyano, -CONHR² und/oder -NHCOR² substituiert sein kann;
einen Rest der Formel -L-R³;
einen Rest der Formel -NR²₂;
R, R' unabhängig voneinander
Wasserstoff;
C₁-C₃₀-Alkyl, dessen Kohlenstoffkette durch eine oder mehrere Gruppierungen -O-, -S-, -NR¹-, -CO- und/oder -SO₂- unterbrochen sein kann und das durch Cyano, C₁-C₆-Alkoxy, Aryl, das durch C₁-C₁₈-Alkyl oder C₁-C₆-Alkoxy substituiert sein kann, und/oder einen über ein Stickstoffatom gebundenen 5- bis 7-gliedrigen heterocyclischen Rest, der weitere Heteroatome enthalten und aromatisch sein kann, ein- oder mehrfach substituiert sein kann;
C₅-C₈-Cycloalkyl, dessen Kohlenstoffgerüst durch eine oder mehrere Gruppierungen -O-, -S- und/oder -NR¹- unterbrochen und/oder das durch C₁-C₆-Alkyl ein- oder mehrfach substituiert sein kann;
Aryl oder Hetaryl, das durch C₁-C₁₈-Alkyl, C₁-C₆-Alkoxy, Cyano, -CONHR², -NHCOR² und/oder Aryl- oder Hetarylazo, das jeweils durch C₁-C₁₀-Alkyl, C₁-C₆-Alkoxy oder Cyano substituiert sein kann, ein- oder mehrfach substituiert sein kann;
L 1,2-Ethylen, 1,2-Ethenylen oder 1,2-Ethinylen;
R¹ Wasserstoff oder C₁-C₆-Alkyl;
R² Wasserstoff; C₁-C₁₈-Alkyl; Aryl oder Hetaryl, das jeweils durch C₁-C₆-Alkyl, C₁-C₆-Alkoxy, Halogen, Hydroxy, Carboxy oder Cyano substituiert sein kann;
R³ C₁-C₁₈-Alkyl, dessen Kohlenstoffkette durch eine oder mehrere Gruppierungen -O-, -S-, -NR¹-, -CO- und/oder -SO₂- unterbrochen sein kann und das durch -COOR¹, -SO₃R¹, Hydroxy, Cyano, C₁-C₆-Alkoxy, Aryl, das durch C₁-C₁₈-Alkyl oder C₁-C₆-Alkoxy substituiert sein kann, und/oder einen über ein Stickstoffatom gebundenen 5-bis 7-gliedrigen heterocyclischen Rest, der weitere Heteroatome enthalten und aromatisch sein kann, ein-oder mehrfach substituiert sein kann.

2. 1,6,9,14-tetrasubstituierte Terrylentetracarbonsäurediimide der Formel I nach Anspruch 1, in der die Variablen folgende Bedeutung haben:
X, Y unabhängig voneinander
Brom; Cyano;
Phenoxy, Phenylthio, Pyridyloxy oder Pyridylthio, das jeweils ein- oder mehrfach durch C₁-C₁₂-Alkyl, C₁-C₁₂-Alkoxy, -COOR¹, Halogen, Hydroxy, Carboxy, Cyano, -CONHR² und/oder -NHCOR² substituiert sein kann;
einen Rest der Formel -L-R³;
R, R' unabhängig voneinander
Wasserstoff;
C₁-C₃₀-Alkyl, dessen Kohlenstoffkette durch eine oder mehrere Gruppierungen -O- und/oder -CO- unterbrochen sein kann und das durch Cyano, C₁-C₆-Alkoxy und/oder Aryl, das durch C₁-C₁₈-Alkyl oder C₁-C₆-Alkoxy substituiert sein kann, ein- oder mehrfach substituiert sein kann;
C₅-C₈-Cycloalkyl, das durch C₁-C₆-Alkyl ein- oder mehrfach substituiert sein kann;
Phenyl, Naphthyl oder Pyridyl, das durch C₁-C₁₈-Alkyl, C₁-C₆-Alkoxy, Cyano, -CONHR² und/oder -NHCOR² ein- oder mehrfach substituiert sein kann;
L 1,2-Ethenylen oder 1,2-Ethinylen;_
_{R}¹ Wasserstoff oder C₁-C₆-Alkyl;
R² Wasserstoff; C₁-C₁₈-Alkyl; Phenyl, das-jeweils durch C₁-C₆-Alkyl, C₁-C₆-Alkoxy, Halogen, Hydroxy, Carboxy oder Cyano substituiert sein kann;
R³ C₁-C₁₈-Alkyl, dessen Kohlenstoffkette durch eine oder mehrere Gruppierungen -O- und/oder -CO- unterbrochen sein kann und das durch -COOR¹, Hydroxy, Cyano, C₁-C₆-Alkoxy und/oder Aryl, das durch C₁-C₁₈-Alkyl oder C₁-C₆-Alkoxy substituiert sein kann, ein- oder mehrfach substituiert sein kann.

3. Verfahren zur Herstellung von 1,6,9,14-tetrasubstituierten Terrylentetracarbonsäurediimiden der allgemeinen Formel Ia 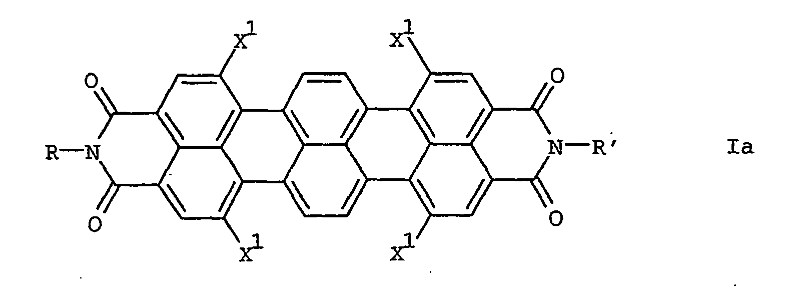 in der die Variablen folgende Bedeutung haben:
X¹ Brom; Aryloxy, Arylthio, Hetaryloxy oder Hetarylthio, das jeweils ein- oder mehrfach durch C₁-C₁₂-Alkyl, C_{I}-C₁₂-Alkoxy, -COOR¹, -S0₃R¹, Halogen, Hydroxy, Carboxy, Cyano, -CONHR² und/oder -NHCOR² substituiert sein kann;
R, R' unabhängig voneinander
Wasserstoff;
C₁-C₃₀-Alkyl, dessen Kohlenstoffkette durch eine oder mehrere Gruppierungen -O-, -S-, -NR¹-, -CO- und/oder -SO₂- unterbrochen sein kann und das durch Cyano, C₁-C₆-Alkoxy, Aryl, das durch C₁-C₁₈-Alkyl oder C₁-C₆-Alkoxy substituiert sein kann, und/oder einen über ein Stickstoffatom gebundenen 5- bis 7-gliedrigen heterocyclischen Rest, der weitere Heteroatome enthalten und aromatisch sein kann, ein- oder mehrfach substituiert sein kann;
C₅-C₈-Cycloalkyl, dessen Kohlenstoffgerüst durch eine oder mehrere Gruppierungen -O-, -S- und/oder -NR¹- unterbrochen und/oder das durch C₁-C₆-Alkyl ein- oder mehrfach substituiert sein kann;
Aryl oder Hetaryl, das durch C₁-C₁₈-Alkyl, C₁-C₆-Alkoxy, Cyano, -CONHR², -NHCOR² und/oder Aryl- oder Hetarylazo, das jeweils durch C₁-C₁₀-Alkyl, C₁-C₆-Alkoxy oder Cyano substituiert sein kann, ein- oder mehrfach substituiert sein kann;
R¹ Wasserstoff oder C₁-C₆-Alkyl;
R² Wasserstoff; C₁-C₁₈-Alkyl; Aryl oder Hetaryl, das jeweils durch C₁-C₆-Alkyl, C₁-C₆-Alkoxy, Halogen, Hydroxy, Carboxy oder Cyano substituiert sein kann,
**dadurch gekennzeichnet, daß** man
a) ein Terrylentetracarbonsäurediimid der allgemeinen Formel II 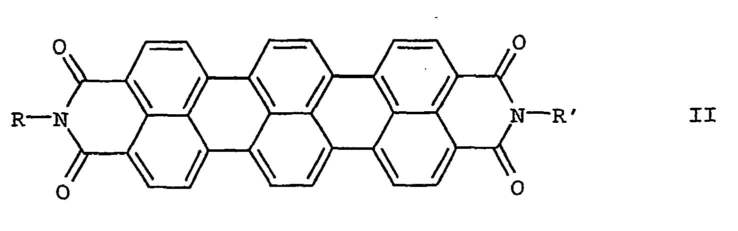 mit elementarem Brom in Gegenwart eines Halogenkohlenwasserstoffs als Lösungsmittel zum 1,6,9,14-Tetrabromterrylentetracarbonsäurediimid der allgemeinen Formel Ia' 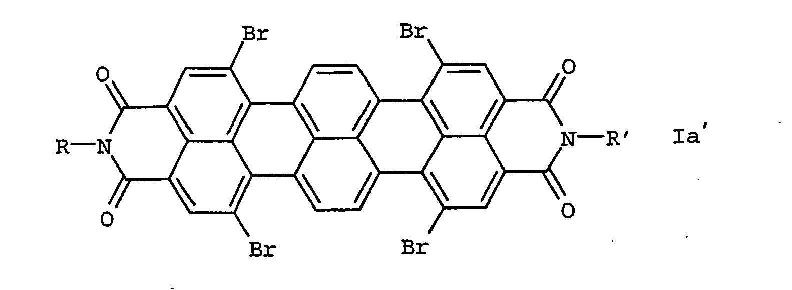 umsetzt und gewünschtenfalls
b) das in Schritt a) erhaltene Tetrabromterrylentetracarbonsäurediimid Ia' in Gegenwart eines inerten stickstoffbasischen Lösungsmittels und einer Base mit einem Alkohol oder Thioalkohol der allgemeinen Formel III mit X¹ ≠ Br 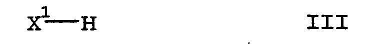 in das 1,6,9,14-tetrasubstituierte Terrylentetracarbonsäurediimid der Formel Ia mit X¹ ≠ Brom überführt.

4. Verfahren zur Herstellung von 1,6,9,14-tetrasubstituierten Terrylentetracarbonsäurediimiden der allgemeinen Formel Ib 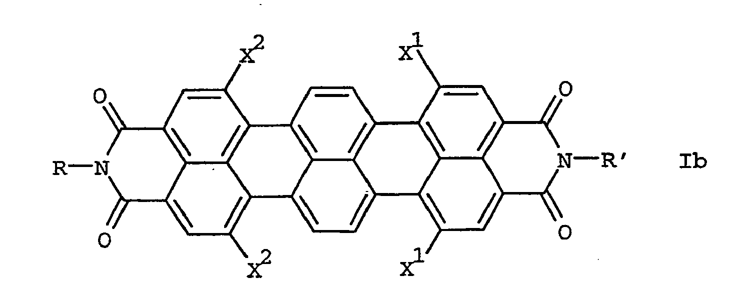 in der die Variablen folgende Bedeutung haben:
X² Aryloxy, Arylthio, Hetaryloxy oder Hetarylthio, das jeweils ein- oder mehrfach durch C₁-C₁₂-Alkyl, C₁-C₁₂-Alkoxy, -COOR¹, -SO₃R¹, Cyano, -CONHR⁴ und/oder -NHCOR⁴ substituiert sein kann;
X¹ Brom; Aryloxy, Arylthio, Hetaryloxy oder Hetarylthio, das jeweils ein- oder mehrfach durch C₁-C₁₂-Alkyl, C₁-C₁₂-Alkoxy, -COOR¹, -SO₃R¹, Halogen, Hydroxy, Carboxy, Cyano, -CONHR² und/oder -NHCOR² substituiert sein kann;
R, R' unabhängig voneinander
Wasserstoff;
C₁-C₃₀-Alkyl, dessen Kohlenstoffkette durch eine oder mehrere Gruppierungen -O-, -S-, -NR¹-, -CO- und/oder -SO₂- unterbrochen sein kann und das durch Cyano, C₁-C₆-Alkoxy, Aryl, das durch C₁-C₁₈-Alkyl oder C₁-C₆-Alkoxy substituiert sein kann, und/oder einen über ein Stickstoffatom gebundenen 5- bis 7-gliedrigen heterocyclischen Rest, der weitere Heteroatome enthalten und aromatisch sein kann, ein- oder mehrfach substituiert sein kann;
C₅-C₈-Cycloalkyl, dessen Kohlenstoffgerüst durch eine oder mehrere Gruppierungen -O-, -S- und/oder -NR¹- unterbrochen und/oder das durch C₁-C₆-Alkyl ein- oder mehrfach substituiert sein kann;
Aryl oder Hetaryl, das durch C₁-C₁₈-Alkyl, C₁-C₆-Alkoxy, Cyano, -CONHR², -NHCOR² und/oder Aryl- oder Hetarylazo, das jeweils durch C₁-C₁₀-Alkyl, C₁-C₆-Alkoxy oder Cyano substituiert sein kann, ein- oder mehrfach substituiert sein kann;
R¹ Wasserstoff oder C₁-C₆-Alkyl;
R² Wasserstoff; C₁-C₁₈-Alkyl; Aryl oder Hetaryl, das jeweils durch C₁-C₆-Alkyl, C₁-C₆-Alkoxy, Halogen, Hydroxy, Carboxy oder Cyano substituiert sein kann,
R⁴ C₁-C₁₈-Alkyl; Aryl oder Hetaryl, das jeweils durch C₁-C₆-Alkyl, C₁-C₆-Alkoxy, Hydroxy oder Cyano substituiert sein kann,
**dadurch gekennzeichnet, daß** man
a) ein 1,6-disubstiutiertes Terrylentetracarbonsäurediimid der allgemeinen Formel IV 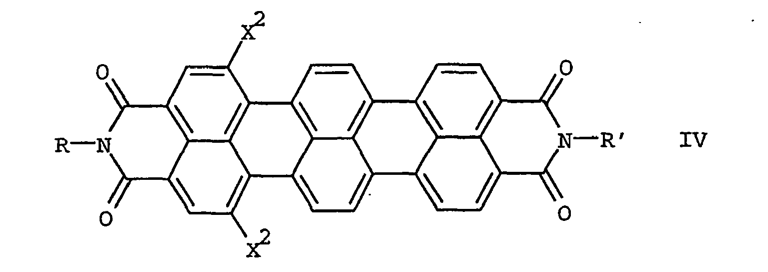 mit elementarem Brom in Gegenwart eines Halogenkohlenwasserstoffs als Lösungsmittel zum 9,14-dibromierten Terrylentetracarbonsäurediimid der allgemeinen Formel Ib' 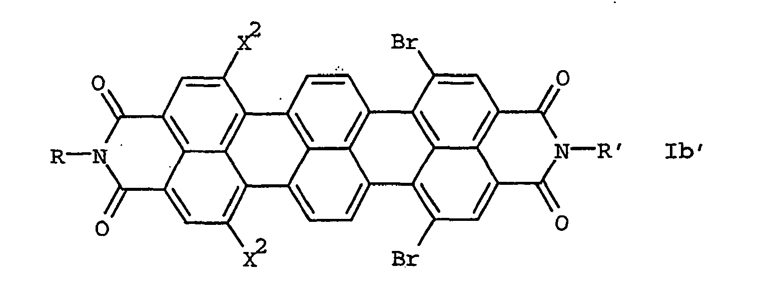 umsetzt und gewünschtenfalls
b) das in Schritt a) erhaltene 9,14-dibromierte Terrylentetracarbonsäurediimid Ib' in Gegenwart eines inerten stickstoffbasischen Lösungsmittels und einer Base mit einem Alkohol oder Thioalkohol der allgemeinen Formel III mit X¹≠ Brom 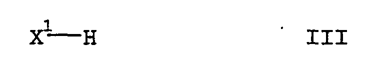 in das 1,6,9,14-tetrasubstituierte Terrylentetracarbonsäurediimid der Formel Ib mit X¹ ≠ Brom überführt.

5. Verfahren zur Herstellung von 1,6,9,14-tetrasubstituierten Terrylentetracarbonsäurediimiden der allgemeinen Formel Ic 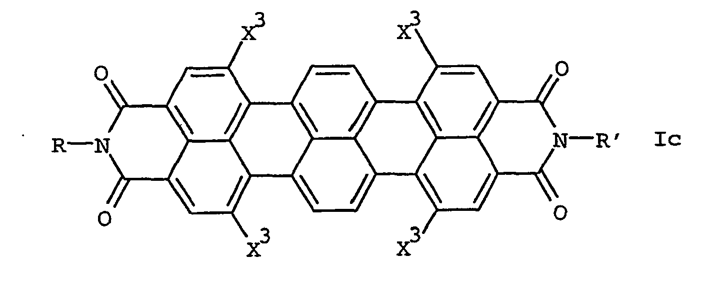 in der die Variablen folgende Bedeutung haben:
X³ einen Rest der Formel -L-R³;
R, R' unabhängig voneinander
Wasserstoff;
C₁-C₃₀-Alkyl, dessen Kohlenstoffkette durch eine oder mehrere Gruppierungen -O-, -S-, -NR¹-, -CO- und/oder -SO₂- unterbrochen sein kann und das durch Cyano, C₁-C₆-Alkoxy, Aryl, das durch C₁-C₁₈-Alkyl oder C₁-C₆-Alkoxy substituiert sein kann, und/oder einen über ein Stickstoffatom gebundenen 5- bis 7-gliedrigen heterocyclischen Rest, der weitere Heteroatome enthalten und aromatisch sein kann, ein- oder mehrfach substituiert sein kann;
C₅-C₈-Cycloalkyl, dessen Kohlenstoffgerüst durch eine oder mehrere Gruppierungen -O-, -S- und/oder -NR¹- unterbrochen und/oder das durch C₁-C₆-Alkyl ein- oder mehrfach substituiert sein kann;
Aryl oder Hetaryl, das durch C₁-C₁₈-Alkyl, C₁-C₆-Alkoxy, Cyano, -CONHR², -NHCOR² und/oder Aryl- oder Hetarylazo, das jeweils durch C₁-C₁₀-Alkyl, C₁-C₆-Alkoxy oder Cyano substituiert sein kann, ein- oder mehrfach substituiert sein kann;
L 1,2-Ethylen, 1,2-Ethenylen oder 1,2-Ethinylen;
R¹ Wasserstoff oder C₁-C₆-Alkyl;
R² Wasserstoff; C₁-C₁₈-Alkyl; Aryl oder Hetaryl, das jeweils durch C₁-C₆-Alkyl, C₁-C₆-Alkoxy, Halogen, Hydroxy, Carboxy oder Cyano substituiert sein kann;
R³ C₁-C₁₈-Alkyl, dessen Kohlenstoffkette durch eine oder mehrere Gruppierungen -O-, -S-, -NR¹-, -CO- und/oder -SO₂- unterbrochen sein kann und das durch -COOR¹, -SO₃R¹, Hydroxy, Cyano, C₁-C₆-Alkoxy, Aryl, das durch C₁-C₁₈-Alkyl oder C₁-C₆-Alkoxy substituiert sein kann, und/oder einen über ein Stickstoffatom gebundenen 5-bis 7-gliedrigen heterocyclischen Rest, der weitere Heteroatome enthalten und aromatisch sein kann, ein-oder mehrfach substituiert sein kann,
**dadurch gekennzeichnet, daß** man ein 1,6,9,14-Tetrabromterrylentetracarbonsäurediimid der allgemeinen Formel Ia' 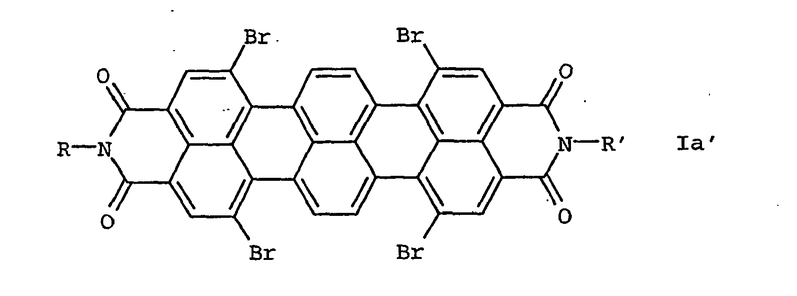 in Gegenwart eines aprotischen Lösungsmittels, eines Palladiumkomplexes als Katalysator, eines Kupfersalzes als Cokatalysator und einer Base mit einem 1-Alkin der allgemeinen Formel V
H-C≡C-R³ V
umsetzt und gewünschtenfalls die in den Resten X³ des erhaltenen 1,6,9,14-tetrasubstituierten Terrylentetracarbonsäurediimids enthaltenen ungesättigten Bindungen zusätzlich reduziert.

6. Verfahren zur Herstellung von 1,6,9,14-tetrasubstituierten Terrylentetracarbonsäurediimiden der allgemeinen Formel Id 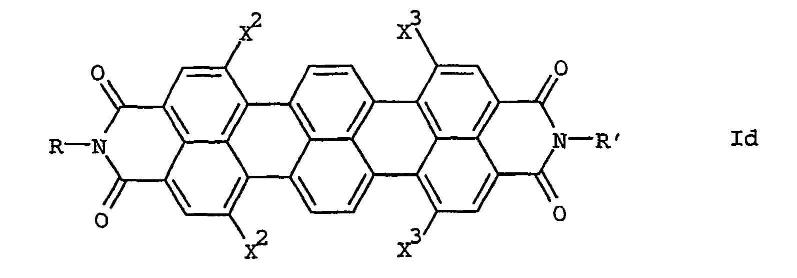 in der die Variablen folgende Bedeutung haben:
X² Aryloxy, Arylthio, Hetaryloxy oder Hetarylthio, das jeweils ein- oder mehrfach durch C₁-C₁₂-Alkyl, C₁-C₁₂-Alkoxy, -COOR¹, -SO₃R¹, Cyano, -CONHR⁴ und/oder -NHCOR⁴ substituiert sein kann;
X³ einen Rest der Formel -L-R³;
R, R' gleich oder verschieden und unabhängig voneinander
Wasserstoff;
C₁-C₃₀-Alkyl, dessen Kohlenstoffkette durch eine oder mehrere Gruppierungen -O-, -S-, -NR¹-, -CO- und/oder -SO₂ unterbrochen sein kann und das durch Cyano, C₁-C₆-Alkoxy, Aryl, das durch C₁-C₁₈-Alkyl oder C₁-C₆-Alkoxy substituiert sein kann, und/oder einen über ein Stickstoffatom gebundenen 5- bis 7-gliedrigen heterocyclischen Rest, der weitere Heteroatome enthalten und aromatisch sein kann, ein- oder mehrfach substituiert sein kann;
C₅-C₈-Cycloalkyl, dessen Kohlenstoffgerüst durch eine oder mehrere Gruppierungen -O-, -S- und/oder -NR¹- unterbrochen und/oder das durch C₁-C₆-Alkyl ein- oder mehrfach substituiert sein kann;
Aryl oder Hetaryl, das durch C₁-C₁₈-Alkyl, C₁-C₆-Alkoxy, Cyano, -CONHR², -NHCOR² und/oder Aryl- oder Hetarylazo, das jeweils durch C₁-C₁₀-Alkyl, C₁-C₆-Alkoxy oder Cyano substituiert sein kann, ein- oder mehrfach substituiert sein kann;
L 1,2-Ethylen, 1,2-Ethenylen oder 1,2-Ethinylen;
R¹ Wasserstoff oder C₁-C₆-Alkyl;
R² Wasserstoff; C₁-C₁₈-Alkyl; Aryl oder Hetaryl, das jeweils durch C₁-C₆-Alkyl, C₁-C₆-Alkoxy, Halogen, Hydroxy, Carboxy oder Cyano substituiert sein kann;
R³ C₁-C₁₈-Alkyl, dessen Kohlenstoffkette durch eine oder mehrere Gruppierungen -O-, -S-, -NR¹-, -CO- und/oder -SO₂- unterbrochen sein kann und das durch -COOR¹, -SO₃R¹, Hydroxy, Cyano, C₁-C₆-Alkoxy, Aryl, das durch C₁-C₁₈-Alkyl oder C₁-C₆-Alkoxy substituiert sein kann, und/oder einen über ein Stickstoffatom gebundenen 5-bis 7-gliedrigen heterocyclischen Rest, der weitere Heteroatome enthalten und aromatisch sein kann, ein-oder mehrfach substituiert sein kann;
R⁴ C₁-C₁₈-Alkyl; Aryl oder Hetaryl, das jeweils durch C₁-C₆-Alkyl, C₁-C₆-Alkoxy, Halogen, Hydroxy, Carboxy oder Cyano substituiert sein kann,
**dadurch gekennzeichnet, daß** man ein 9,14-dibromiertes Terrylentetracarbonsäurediimid der allgemeinen Formel Ib' 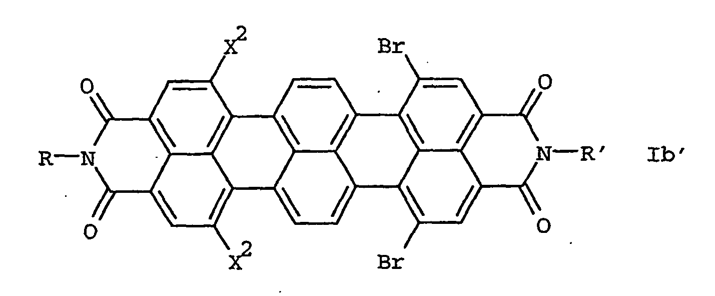 in Gegenwart eines aprotischen Lösungsmittels, eines Palladiumkomplexes als Katalysator, eines Kupfersalzes als Cokatalysator und einer Base mit einem 1-Alkin der allgemeinen Formel V
H-C≡C-R³ V
umsetzt und gewünschtenfalls die in den Resten X³ des erhaltenen 1,6,9,14-tetrasubstituierten Terrylentetracarbonsäurediimids enthaltenen ungesättigten Bindungen zusätzlich reduziert.

7. Verwendung von 1,6,9,14-tetrasubstituierten Terrylentetracarbonsäurediimiden der Formel 1 gemäß Anspruch 1 oder 2 zur Einfärbung von hochmolekularen organischen und anorganischen Materialien.

8. Verwendung von 1,6,9,14-tetrasubstituierten Terrylentetracarbonsäurediimiden der Formel 1 gemäß Anspruch 1 oder 2 als Dispergierhilfsmittel, Pigmentadditive für organische Pigmente und Zwischenprodukte für die Herstellung von Fluoreszenzfarbmitteln und Pigmentadditiven.

9. Verwendung von 1,6,9,14-tetrasubstituierten Terrylentetracarbonsäurediimiden der Formel 1 gemäß Anspruch 1 oder 2 zur Herstellung farbiger oder im nahinfraroten Bereich des elektromagnetischen Spektrums absorbierender und/oder emittierender wäßriger Polymerisatdispersionen.

10. Verwendung von 1,6,9,14-tetrasubstituierten Terrylentetracarbonsäurediimiden der Formel 1 gemäß Anspruch 1 oder 2 als Emitter in Elektro- und Chemilumineszenzanwendungen, als Aktivkomponenten in der Fluoreszenzkonversion, in Biolumineszenzarrays und in der Photovoltaik und als Laserfarbstoffe.

## Claims

1. A 1,6,9,14-tetrasubstituted terrylenetetracarboxylic diimide of the general formula I 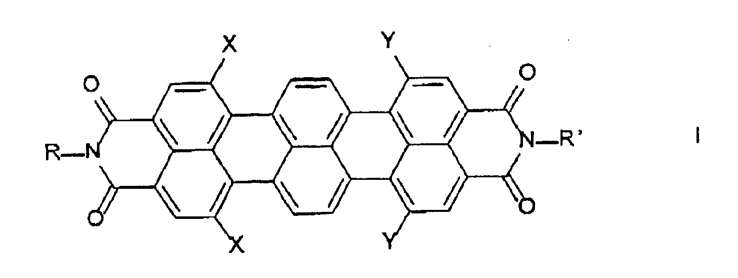 in which the variables are defined as follows:
X and Y are each independently
bromine; cyano;
aryloxy, arylthio, hetaryloxy or hetarylthio, each of which may be mono- or polysubstituted by C₁-C₁₂-alkyl, C₁-C₁₂-alkoxy, -COOR¹, -SO₃R¹, halogen, hydroxyl, carboxyl, cyano, -CONHR² and/or -NHCOR²;
a radical of the formula -L-R³;
a radical of the formula -NR²₂;
R and R' are each independently
hydrogen;
C₁-C₃₀-alkyl whose carbon chain may be interrupted by one or more -O-, -S-, -NR¹-, -CO- and/or -SO₂- groups, and which may be mono- or polysubstituted by cyano, C₁-C₆-alkoxy, aryl which may be substituted by C₁-C₁₈-alkyl or C₁-C₆-alkoxy, and/or a 5- to 7-membered heterocyclic radical which is bonded via a nitrogen atom and may comprise further heteroatoms and be aromatic;
C₅-C₈-cycloalkyl whose carbon skeleton may be interrupted by one or more -O-, -S- and/or -NR¹- groups, and/or which may be mono- or polysubstituted by C₁-C₆-alkyl;
aryl or hetaryl, each of which may be mono- or polysubstituted by C₁-C₁₈-alkyl, C₁-C₆-alkoxy, cyano, -CONHR², -NHCOR² and/or aryl or hetarylazo, each of which may be substituted by C₁-C₁₀-alkyl, C₁-C₆-alkoxy or cyano;
L is 1,2-ethylene, 1,2-ethenylene or 1,2-ethynylene;
R¹ is hydrogen or C₁-C₆-alkyl;
R² is hydrogen; C₁-C₁₈-alkyl; aryl or hetaryl, each of which may be substituted by C₁-C₆-alkyl, C₁-C₆-alkoxy, halogen, hydroxyl, carboxyl or cyano;
R³ is C₁-C₁₈-alkyl whose carbon chain may be interrupted by one or more -O-, -S-, -NR¹-, -CO- and/or -SO₂- groups, and may be mono- or polysubstituted by -COOR¹, -SO₃R¹, hydroxyl, cyano, C₁-C₆-alkoxy, aryl which may be substituted by C₁-C₁₈-alkyl or C₁-C₆-alkoxy, and/or a 5- to 7-membered heterocyclic radical which is bonded via a nitrogen atom and may comprise further heteroatoms and be aromatic,
with the proviso that N,N'-bis(2,6-diisopropylphenyl)-1,6,9,14-tetrabromoterrylene-3,4:11,12-tetracarboxylic diimide, N,N'-bis(2,6-diisopropylphenyl)-1,6,9,14-tetra(p-tert-octylphenoxy)terrylene-3,4:11,12-tetracarboxylic diimide, N-cyclohexyl-N'-(2,6-diisopropylphenyl)-1,6,9,14-tetrabromoterrylene-3,4:11,12- tetracarboxylic diimide, N-cyclohexyl-N'-(2,6-diisopropylphenyl)-1,6,9,14-tetra(p-tert-octylphenoxy)terrylene-3,4:11,12-tetracarboxylic diimide are excluded.

2. A 1,6,9,14-tetrasubstituted terrylenetetracarboxylic diimide of the formula I as claimed in claim 1, in which the variables are defined as follows:
X and Y are each independently
bromine; cyano;
phenoxy, phenylthio, pyridyloxy or pyridylthio, each of which may be mono- or polysubstituted by C₁-C₁₂-alkyl, C₁-C₁₂-alkoxy, -COOR¹, halogen, hydroxyl, carboxyl, cyano, -CONHR² and/or-NHCOR² ;
a radical of the formula -L-R³;
R and R' are each independently
hydrogen;
C₁-C₃₀-alkyl whose carbon chain may be interrupted by one or more -O- and/or -CO- groups, and which may be mono- or polysubstituted by cyano, C₁-C₆-alkoxy and/or aryl which may be substituted by C₁-C₁₈-alkyl or C₁-C₆-alkoxy;
C₅-C₈-cycloalkyl, each of which may be mono- or polysubstituted by C₁-C₆-alkyl;
phenyl, naphthyl or pyridyl which may be mono- or polysubstituted by C₁-C₁₈-alkyl, C₁-C₆-alkoxy, cyano, -CONHR² and/or -NHCOR²;
L is 1,2-ethenylene or 1,2-ethynylene;
R¹ is hydrogen or C₁-C₆-alkyl ;
R² is hydrogen; C₁-C₁₈-alkyl; phenyl which may be substituted by C₁-C₆-alkyl, C₁-C₆-alkoxy, halogen, hydroxyl, carboxyl or cyano;
R³ is C₁-C₁₈-alkyl whose carbon chain may be interrupted by one or more -O- and/or -CO- groups, and which may be mono- or polysubstituted by -COOR¹, hydroxyl, cyano, C₁-C₆-alkoxy and/or aryl which may be substituted by C₁-C₁₈-alkyl or C₁-C₆-alkoxy,
with the proviso that N,N'-bis(2,6-diisopropylphenyl)-1,6,9,14-tetrabromoterrylene-3,4:11,12-tetracarboxylic diimide, N,N'-bis(2,6-diisopropylphenyl)-1,6,9,14-tetra(p-tert-octylphenoxy)terrylene-3,4:11,12-tetracarboxylic diimide, N-cyclohexyl-N'-(2,6-diisopropylphenyl)-1,6,9,14-tetrabromoterrylene-3,4:11,12-tetracarboxylic diimide, N-cyclohexyl-N'-(2,6-diisopropylphenyl)-1,6,9,14-tetra(p-tert-octylphenoxy)terrylene-3,4:11,12-tetracarboxylic diimide are excluded.

3. A process for preparing 1,6,9,14-tetrasubstituted terrylenetetracarboxylic diimides of the general formula Ia 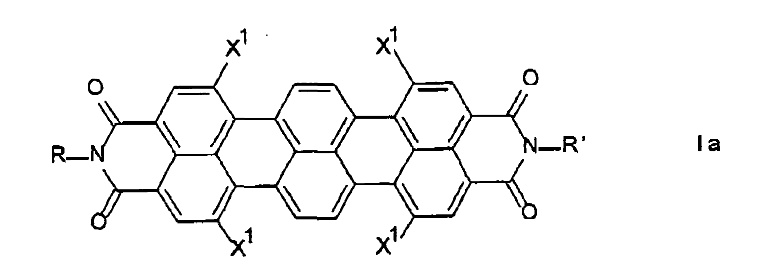 in which the variables are defined as follows:
X¹ is bromine; aryloxy, arylthio, hetaryloxy or hetarylthio, each of which may be mono- or polysubstituted by C₁-C₁₂-alkyl, C₁-C₁₂-alkoxy,-COOR¹, -SO₃R¹, halogen, hydroxyl, carboxyl, cyano, -CONHR² and/or -NHCOR²;
R and R' are each independently
hydrogen;
C₁-C₃₀-alkyl whose carbon chain may be interrupted by one or more -O-, -S-, -NR¹-, -CO-and/or -SO₂- groups, and which may be mono- or polysubstituted by cyano, C₁-C₆-alkoxy, aryl which may be substituted by C₁-C₁₈-alkyl or C₁-C₆-alkoxy, and/or a 5- to 7-membered heterocyclic radical which is bonded via a nitrogen atom and may comprise further heteroatoms and be aromatic;
C₅-C₈-CYCloalkyl whose carbon skeleton may be interrupted by one or more -O-, -S- and/or -NR¹- groups, and/or which may be mono- or polysubstituted by C₁-C₆-alkyl;
aryl or hetaryl, each of which may be mono- or polysubstituted by C₁-C₁₈-alkyl, C₁-C₆-alkoxy, cyano, -CONHR², -NHCOR² and/or aryl or hetarylazo, each of which may be substituted by C₁-C₁₀-alkyl, C₁-C₆-alkoxy or cyano;
R¹ is hydrogen or C₁-C₆-alkyl;
R² is hydrogen; C₁-C₁₈-alkyl; aryl or hetaryl, each of which may be substituted by C₁-C₆-alkyl, C₁-C₆-alkoxy, halogen, hydroxyl, carboxyl or cyano,
with the proviso that N,N'-bis(2,6-diisopropylphenyl)-1,6,9,14-tetrabromoterrylene-3,4:11,12-tetracarboxylic diimide, N,N'-bis(2,6-diisopropylphenyl)-1,6,9,14-tetra(p-tert-octylphenoxy)terrylene-3,4:11,12-tetracarboxylic diimide, N-cyclohexyl-N'-(2,6-diisopropylphenyl)-1,6,9,14-tetrabromoterrylene-3,4:11,12-tetracarboxylic diimide, N-cyclohexyl-N'-(2,6-diisopropylphenyl)-1,6,9,14-tetra(p-tert-octylphenoxy)terrylene-3,4:11,12- tetracarboxylic diimide are excluded,
which comprises
a) reacting a terrylenetetracarboxylic diimide of the general formula II 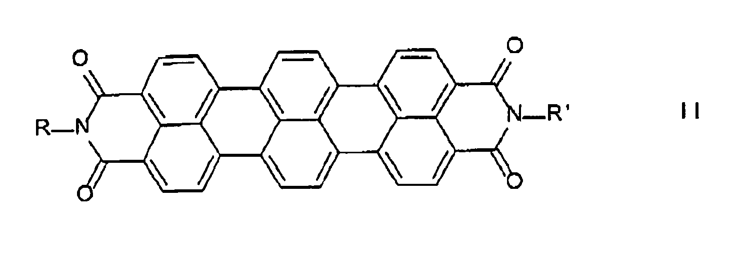 with elemental bromine in the presence of a halohydrocarbon as solvent to give the 1,6,9,14-tetrabromoterrylenetetracarboxylic diimide of the general formula Ia' 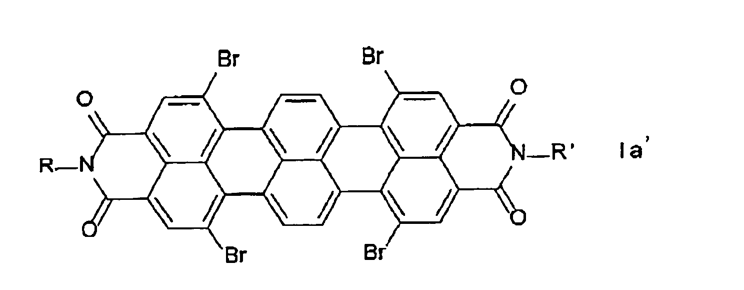 and, if desired,
b) converting the tetrabromoterrylenetetracarboxylic diimide Ia' obtained in step a) in the presence of an inert nitrogen-basic solvent and a base with an alcohol or thioalcohol of the general formula III where X¹ ≠ Br 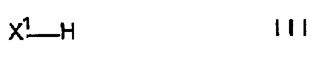 to the 1,6,9,14-tetrasubstituted terrylenetetracarboxylic diimide of the formula Ia where X¹ ≠ bromine, with the proviso that N,N'-bis(2,6-diisopropylphenyl)-1,6,9,14-tetra(p-tert-octylphenoxy)terrylene-3,4:11,12-dicarboxylic diimide and N-cyclohexyl-N'-(2,6-diisopropylphenyl)-1,6,9,14-tetra(p-tert-octylphenoxy)terrylene-3,4:11,12-dicarboxylic diimide are excluded.

4. A process for preparing 1,6,9,14-tetrasubstituted terrylenetetracarboxylic dimides of the general formula Ib 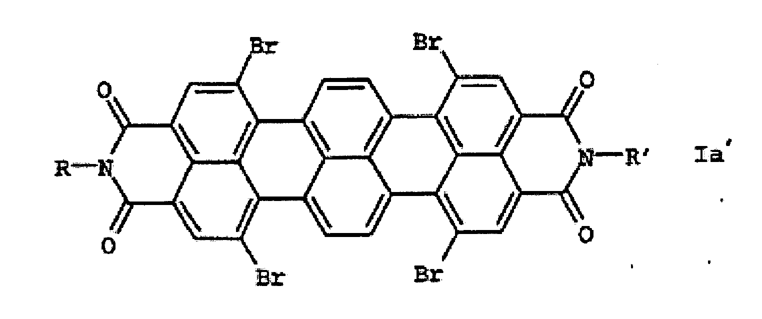 in which the variables are defined as follows:
x² is aryloxy, arylthio, hetaryloxy or hetarylthio, each of which may be mono- or polysubstituted by C₁-C₁₂-alkyl, C₁-C₁₂-alkoxy,-COOR¹, -SO₃R¹, cyano, -CONHR⁴ and/or -NHCOR⁴;
X¹ is bromine; aryloxy, arylthio, hetaryloxy or hetarylthio, each of which may be mono- or polysubstituted by C₁-C₁₂-alkyl, C₁-C₁₂-alkoxy,-COOR¹, -S0₃R¹, halogen, hydroxyl, carboxyl, cyano, -CONHR² and/or -NHCOR²;
R and R' are each independently
hydrogen;
C₁-C₃₀-alkyl whose carbon chain may be interrupted by one or more -0-, -S-, -NR¹-, -CO- and/or -SO₂- groups, and which may be mono- or polysubstituted by cyano, C₁-C₆-alkoxy, aryl which may be substituted by C₁-C₁₈-alkyl or C₁-C₆-alkoxy, and/or a 5- to 7-membered heterocyclic radical which is bonded via a nitrogen atom and may comprise further heteroatoms and be aromatic; C₅-C₈-cycloalkyl whose carbon skeleton may be interrupted by one or more -O-, -S- and/or -NR¹- groups, and/or which may be mono- or polysubstituted by C₁-C₆-alkyl;
aryl or hetaryl, each of which may be mono- or polysubstituted by C₁-C₁₈-alkyl, C₁-C₆-alkoxy, cyano, -CONHR², -NHCOR² and/or aryl or hetarylazo, each of which may be substituted by C₁-C₁₀-alkyl, C₁-C₆-alkoxy or cyano;
R¹ is hydrogen or C₁-C₆-alkyl;
R² is hydrogen; C₁-C₁₈-alkyl; aryl or hetaryl, each of which may be substituted by C₁-C₆-alkyl, C₁-C₆-alkoxy, halogen, hydroxyl, carboxyl or cyano,
R⁴ is C₁-C₁₈-alkyl; aryl or hetaryl, each of which may be substituted by C₁-C₆-alkyl, C₁-C₆-alkoxy, hydroxyl or cyano,
which comprises
a) reacting a 1,6-disubstituted terrylenetetracarboxylic diimide of the general formula IV 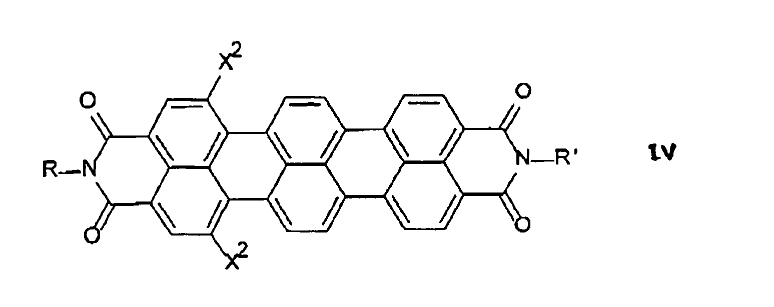 with elemental bromine in the presence of a halohydrocarbon as solvent to give the 9,14-dibrominated terrylenetetracarboxylic diimide of the general formula Ib' 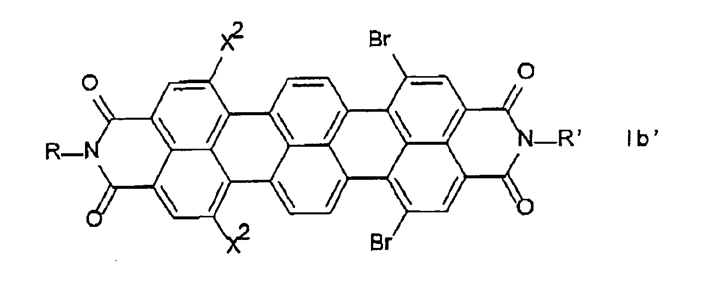 and, if desired,
b) converting the 9,14-dibrominated terrylenetetracarboxylic diimide Ib' obtained in step a) in the presence of an inert nitrogen-basic solvent and a base with an alcohol or thioalcohol of the general formula III where X¹ # bromine 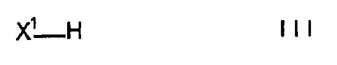 to the 1,6,9,14-tetrasubstituted terrylenetetracarboxylic diimide of the formula Ib where X¹ ≠ bromine.

5. A process for preparing 1,6,9,14-tetrasubstituted terrylenetetracarboxylic diimides of the general formula Ic 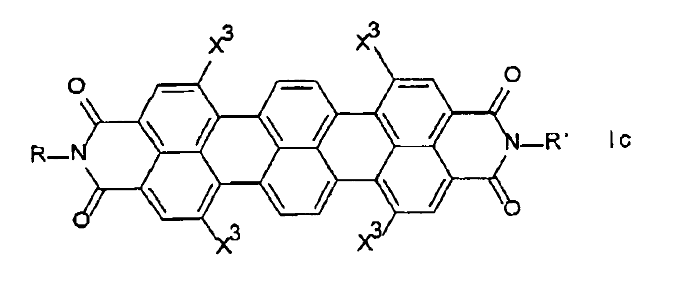 in which the variables are defined as follows:
X³ is a radical of the formula -L-R³;
R and R' are each independently
hydrogen;
C₁-C₃₀-alkyl whose carbon chain may be interrupted by one or more -O-, -S-, -NR¹-, -CO- and/or -SO₂- groups, and which may be mono- or polysubstituted by cyano, C₁-C₆-alkoxy, aryl which may be substituted by C₁-C₁₈-alkyl or C₁-C₆-alkoxy, and/or a 5- to 7-membered heterocyclic radical which is bonded via a nitrogen atom and may comprise further heteroatoms and be aromatic;
C₅-C₈-cycloalkyl whose carbon skeleton may be interrupted by one or more -O-, -S- and/or -NR¹- groups, and/or which may be mono- or polysubstituted by C₁-C₆-alkyl;
aryl or hetaryl, each of which may be mono- or polysubstituted by C₁-C₁₈-alkyl, C₁-C₆-alkoxy, cyano, -CONHR², -NHCOR² and/or aryl or hetarylazo, each of which may be substituted by C₁-C₁₀-alkyl, C₁-C₆-alkoxy or cyano;
L is 1,2-ethylene, 1,2-ethenylene or 1,2-ethynylene;
R¹ is hydrogen or C₁-C₆-alkyl ;
R² is hydrogen; C₁-C₁₈-alkyl; aryl or hetaryl, each of which may be substituted by C₁-C₆-alkyl, C₁-C₆-alkoxy, halogen, hydroxyl, carboxyl or cyano;
R³ is C₁-C₁₈-alkyl whose carbon chain may be interrupted by one or more -O-, -S-, -NR¹-, -CO- and/or -SO₂- groups, and may be mono- or polysubstituted by -COOR¹, -SO₃R¹, hydroxyl, cyano, C₁-C₆-alkoxy, aryl which may be substituted by C₁-C₁₈-alkyl or C₁-C₆-alkoxy, and/or a 5- to 7-membered heterocyclic radical which is bonded via a nitrogen atom and may comprise further heteroatoms and be aromatic,
which comprises reacting a 1,6,9,14-tetrabromoterrylenetetracarboxylic diimide of the general formula Ia' 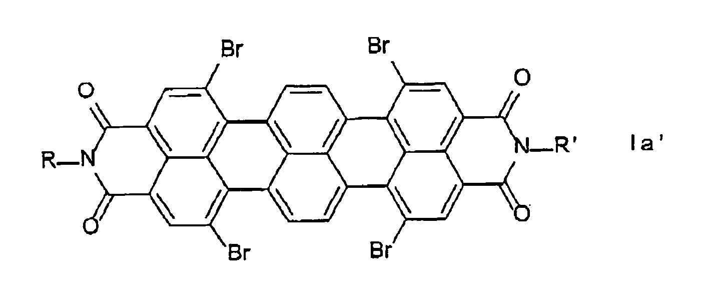 in the presence of an aprotic solvent, a palladium complex as a catalyst, a copper salt as a cocatalyst and a base with a 1-alkyne of the general formula V
H-C≡C-R³ V
and, if desired, additionally reducing the unsaturated bonds present in the X³ radicals of the 1,6,9,14-tetrasubstituted terrylenetetracarboxylic diimide obtained.

6. A process for preparing 1,6,9,14-tetrasubstituted terrylenetetracarboxylic diimides of the general formula Id 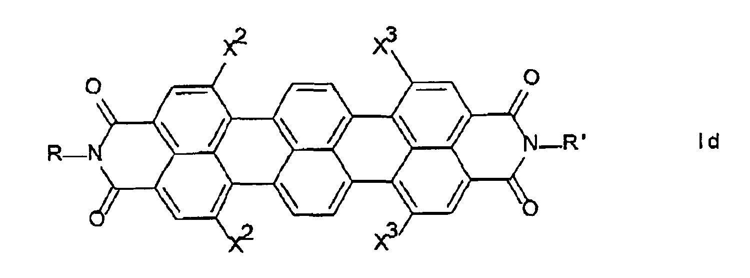 in which the variables are defined as follows:
X² is aryloxy, arylthio, hetaryloxy or hetarylthio, each of which may be mono- or polysubstituted by C₁-C₁₂-alkyl, C₁-C₁₂-alkoxy,-COOR¹, -SO₃R¹, cyano, -CONHR⁴ and/or -NHCOR⁴ ;
X³ is a radical of the formula -L-R³;
R and R' are identical or different and are each independently
hydrogen;
C₁-C₃₀-alkyl whose carbon chain may be interrupted by one or more -O-, -S-, -NR¹-, -CO- and/or -SO₂- groups, and which may be mono- or polysubstituted by cyano, C₁-C₆-alkoxy, aryl which may be substituted by C₁-C₁₈-alkyl or C₁-C₆-alkoxy, and/or a 5- to 7-membered heterocyclic radical which is bonded via a nitrogen atom and may comprise further heteroatoms and be aromatic;
C₅-C₈-cycloalkyl whose carbon skeleton may be interrupted by one or more -O-, -S- and/or -NR¹- groups, and/or which may be mono- or polysubstituted by C₁-C₆-alkyl;
aryl or hetaryl, each of which may be mono- or polysubstituted by C₁-C₁₈-alkyl, C₁-C₆-alkoxy, cyano, -CONHR², -NHCOR² and/or aryl or hetarylazo, each of which may be substituted by C₁-C₁₀-alkyl, C₁-C₆-alkoxy or cyano;
L is 1,2-ethylene, 1,2-ethenylene or 1,2-ethynylene;
R¹ is hydrogen or C₁-C₆-alkyl ;
R² is hydrogen; C₁-C₁₈-alkyl; aryl or hetaryl, each of which may be substituted by C₁-C₆-alkyl, C₁-C₆-alkoxy, halogen, hydroxyl, carboxyl or cyano;
R³ is C₁-C₁₈-alkyl whose carbon chain may be interrupted by one or more -O-, -S-, -NR¹-, -CO- and/or -SO₂- groups, and may be mono- or polysubstituted by -COOR¹, -SO₃R¹, hydroxyl, cyano, C₁-C₆-alkoxy, aryl which may be substituted by C₁-C₁₈-alkyl or C₁-C₆-alkoxy, and/or a 5- to 7-membered heterocyclic radical which is bonded via a nitrogen atom and may comprise further heteroatoms and be aromatic;
R⁴ is C₁-C₁₈-alkyl; aryl or hetaryl, each of which may be substituted by C₁-C₆-alkyl, C₁-C₆-alkoxy, halogen, hydroxyl, carboxyl or cyano,
which comprises reacting a 9,14-dibrominated terrylenetetracarboxylic diimide of the general formula Ib' 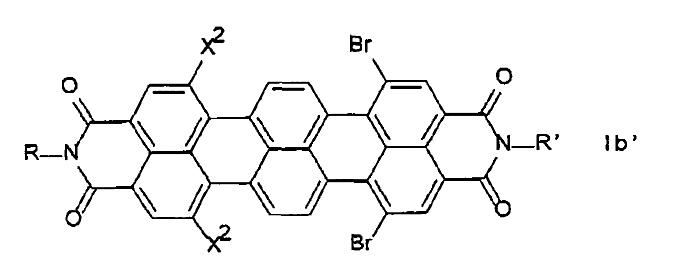 in the presence of an aprotic solvent, a palladium complex as a catalyst, a copper salt as a cocatalyst and a base with a 1-alkyne of the general formula V
H-C≡C-R³ V
and, if desired, additionally reducing the unsaturated bonds present in the X³ radicals of the 1,6,9,14-tetrasubstituted terrylenetetracarboxylic diimide obtained.

7. The use of 1,6,9,14-tetrasubstituted terrylenetetracarboxylic diimides of the formula I as claimed in claim 1 or 2 for coloring high molecular weight organic and inorganic materials.

8. The use of 1,6,9,14-tetrasubstituted terrylenetetracarboxylic diimides of the formula I as claimed in claim 1 or 2 as dispersing aids, pigment additives for organic pigments and intermediates for preparing fluorescent colorants and pigment additives.

9. The use of 1,6,9,14-tetrasubstituted terrylenetetracarboxylic diimides of the formula I as claimed in claim 1 or 2 for preparing aqueous polymer dispersions which are colored or absorb and/or emit in the near infrared region of the electromagnetic spectrum.

10. The use of 1,6,9,14-tetrasubstituted terrylenetetracarboxylic diimides of the formula I as claimed in claim 1 or 2 as emitters in electroluminescence and chemiluminescence applications, as active components in fluorescence conversion, in bioluminescence arrays and in photovoltaics, and as laser dyes.

## Revendications

1. Diimides d'acide terrylènetétracarboxylique 1,6,9,14-tétrasubstitués de formule générale I : 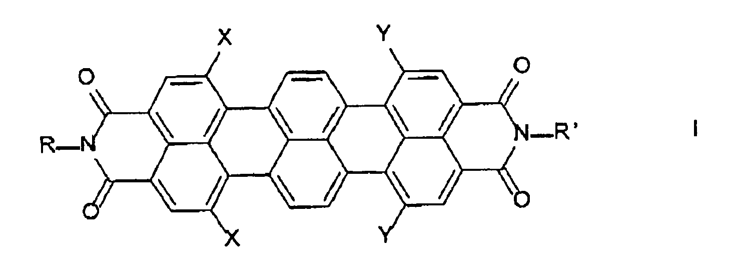 dans laquelle les variables ont la signification suivante :
X, Y indépendamment l'un de l'autre brome ; cyano ;
aryloxy, arylthio, hétaryloxy ou hétarylthio,' qui peut être substitué respectivement une ou plusieurs fois par alkyle en C₁-C₁₂, alcoxy en C₁-C₁₂, -COOR¹ , -SO₃R¹, halogène, hydroxy, carboxy, cyano, -CONHR² et/ou -NHCOR² ;
un radical de formule -L-R³ ;
un radical de formule -NR²₂ ;
R, R' indépendamment l'un de l'autre
hydrogène ;
alkyle en C₁-C₃₀, dont la chaîne de carbone peut être interrompue par un ou plusieurs groupements -O-, -S-, -NR¹-, -CO- et/ou -SO₂- et qui peut être substitué une ou plusieurs fois par cyano, alcoxy en C₁-C₆, aryle, qui peut être substitué par alkyle en C₁-C₁₈ ou alcoxy en C₁-C₆, et/ou un radical hétérocyclique pentagonal à heptagonal, lié par un atome d'azote, qui peut contenir d'autres hétéroatomes et être aromatique ;
cycloalkyle en C₅-C₈, dont le squelette de carbone peut être interrompu par un ou plusieurs groupements -O-, -S- et/ou -NR¹- et/ou qui peut être substitué une ou plusieurs fois par alkyle en C₁-C₆ ;
aryle ou hétaryle, qui peut être substitué une ou plusieurs fois respectivement par alkyle en C₁-C₁₈, alcoxy en C₁-C₆, cyano, -CONHR², -NHCOR² et/ou arylazo ou hétarylazo, qui peut être substitué respectivement par alkyle en C₁-C₁₀, alcoxy en C₁-C₆ ou cyano ;
L 1,2-éthylène, 1,2-éthénylène ou 1,2-éthinylène ;
R¹ hydrogène ou alkyle en C₁-C₆ ;
R² hydrogène ; alkyle en C₁-C₁₈ ; aryle ou hétaryle, qui peut être substitué respectivement par alkyle en C₁-C₆, alcoxy en C₁-C₆, halogène, hydroxy, carboxy ou cyano ;
R³ alkyle en C₁-C₁₈, dont la chaîne de carbone peut être interrompue par un ou plusieurs groupements -O-, -S-, -NR¹-, -CO- et/ou -SO₂- et qui peut être substitué une ou plusieurs fois par -COOR¹, -SO₃R¹, hydroxy, cyano, alcoxy en C₁-C₆, aryle, qui peut être substitué par alkyle en C₁-C₁₈ ou alcoxy en C₁-C₆, et/ou un radical hétérocyclique pentagonal à heptagonal, lié par un atome d'azote, qui peut contenir d'autres hétéroatomes et être aromatique,
sous réserve que le diimide d'acide N,N'-bis(2,6-diisopropylphényl)-1,6,9,14-tétrabromoterrylène-3,4:11,12-tétracarboxylique, le diimide d'acide N,N'-bis(2,6-diisopropylphényl)-1,6,9,14-tétra(p-tert-octylphénoxy)terrylène-3,4:11,12-tétracarboxylique, le diimide d'acide N-cyclohexyl-N'-(2,6-diisopropylphényl)-1,6,9,14-tétrabromoterrylène-3,4:11,12-tétracarboxylique, le diimide d'acide N-cyclohexyl-N'-(2,6-diisopropylphényl)-1,6,9,14-tétra(p-tert-octylphénoxy)terrylène-3,4:11,12-tétracarboxylique soient exclus.

2. Diimides d'acide terrylènetétracarboxylique 1,6,9,14-tétrasubstitués de formule générale I selon la revendication 1, dans laquelle les variables ont la signification suivante :
X, Y indépendamment l'un de l'autre
brome ; cyano ;
phénoxy, phénylthio, pyridyloxy ou pyridylthio, qui peut être substitué respectivement une ou plusieurs fois par alkyle en C₁-C₁₂, alcoxy en C₁-C₁₂, -COOR¹, halogène, hydroxy, carboxy, cyano, -CONHR² et/ou -NHCOR² ;
un radical de formule -L-R³ ;
R, R' indépendamment l'un de l'autre
hydrogène ;
alkyle en C₁-C₃₀, dont la chaîne de carbone peut être interrompue par un ou plusieurs groupements -O- et/ou -CO- et qui peut être substitué une ou plusieurs fois par cyano, alcoxy en C₁-C₆ et/ou aryle, qui peut être substitué par alkyle en C₁-C₁₈ ou alcoxy en C₁-C₆;
cycloalkyle en C₅-C₈, qui peut être substitué une ou plusieurs fois respectivement par alkyle en C₁-C₆ ;
phényle, naphtyle ou pyridyle, qui peut être substitué une ou plusieurs fois par alkyle en C₁-C₁₈, alcoxy en C₁-C₆, cyano, -CONHR² et/ou-NHCOR² ;
L 1,2-éthénylène ou 1,2-éthinylène ;
R¹ hydrogène ou alkyle en C₁-C₆ ;
R² hydrogène ; alkyle en C₁-C₁₈ ; phényle, qui peut être substitué respectivement par alkyle en C₁-C₆, alcoxy en C₁-C₆, halogène, hydroxy, carboxy ou cyano ;
R³ alkyle en C₁-C₁₈, dont la chaîne de carbone peut être interrompue par un ou plusieurs groupements -O- et/ou -CO- et qui peut être substitué une ou plusieurs fois par -COOR¹, hydroxy, cyano, alcoxy en C₁-C₆ et/ou aryle, qui peut être substitué par alkyle en C₁-C₁₈ ou alcoxy en C₁-C₆
sous réserve que le diimide d'acide N,N'-bis(2,6-diisopropylphényl)-1,6,9,14-tétrabromoterrylène-3,4:11,12-tétracarboxylique, le diimide d'acide N,N'-bis(2,6-diisopropylphényl)-1,6,9,14-tétra(p-tert-octylphénoxy)terrylène-3,4:11,12-tétracarboxylique, le diimide d'acide N-cyclohexyl-N'-(2,6-diisopropylphényl)-1,6,9,14-tétrabromoterrylène-3,4:11,12-tétracarboxylique, le diimide d'acide N-cyclohexyl-N'-(2,6-diisopropylphényl)-1,6,9,14-tétra(p-tert-octylphénoxy)terrylène-3,4:11,12-tétracarboxylique soient exclus.

3. Procédé de préparation de diimides d'acide terrylènetétracarboxylique 1,6,9,14-tétrasubstitués de formule générale Ia : 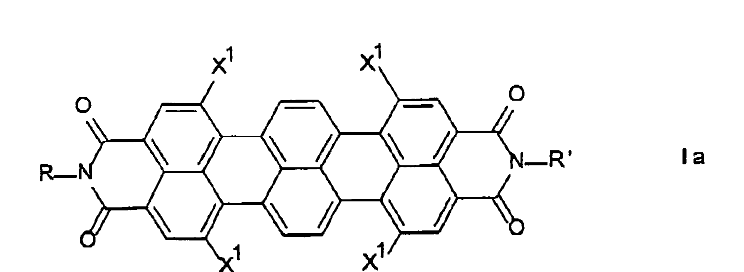 dans laquelle les variables ont la signification suivante :
X¹ brome ; aryloxy, arylthio, hétaryloxy ou hétarylthio, qui peut être substitué respectivement une ou plusieurs fois par alkyle en C₁-C₁₂, alcoxy en C₁-C₁₂, -COOR¹, -SO₃R¹, halogène, hydroxy, carboxy, cyano, -CONHR² et/ou -NHCOR² ;
R, R' indépendamment l'un de l'autre
hydrogène ;
alkyle en C₁-C₃₀, dont la chaîne de carbone peut être interrompue par un ou plusieurs groupements -O-, -S-, -NR¹-, -CO- et/ou -SO₂- et qui peut être substitué une ou plusieurs fois par cyano, alcoxy en C₁-C₆, aryle, qui peut être substitué par alkyle en C₁-C₁₈ ou alcoxy en C₁-C₆, et/ou un radical hétérocyclique pentagonal à heptagonal lié par un atome d'azote, qui peut contenir d'autres hétéroatomes et être aromatique ;
cycloalkyle en C₅-C₈, dont le squelette de carbone peut être interrompu par un ou plusieurs groupements -O-, -S- et/ou -NR¹- et/ou qui peut être substitué une ou plusieurs fois par alkyle en C₁-C₆ ;
aryle ou hétaryle, qui peut être substitué une ou plusieurs fois par alkyle en C₁-C₁₈, alcoxy en C₁-C₆, cyano, -CONHR², -NHCOR² et/ou arylazo ou hétarylazo, qui peut être substitué respectivement par alkyle en C₁-C₁₀, alcoxy en C₁-C₆ ou cyano ;
R¹ hydrogène ou alkyle en C₁-C₆ ;
R² hydrogène ; alkyle en C₁-C₁₈ ; aryle ou hétaryle, qui peut être substitué respectivement par alkyle en C₁-C₆, alcoxy en C₁-C₆, halogène, hydroxy, carboxy ou cyano,
sous réserve que le diimide d'acide N,N'-bis(2,6-diisopropylphényl)-1,6,9,14-tétrabromoterrylène-3,4:11,12-tétracarboxylique, le diimide d'acide N,N'-bis(2,6-diisopropylphényl)-1,6,9,14-tétra(p-tert-octylphénoxy)terrylène-3,4:11,12-tétracarboxylique, le diimide d'acide N-cyclohexyl-N'-(2,6-diisopropylphényl)-1,6,9,14-tétrabromoterrylène-3,4:11,12-tétracarboxylique, le diimide d'acide N-cyclohexyl-N'-(2,6-diisopropylphényl)-1,6,9,14-tétra(p-tert-octylphénoxy)terrylène-3,4:11,12-tétracarboxylique soient exclus,
**caractérisé en ce que** :
a) un diimide d'acide terrylènetétracarboxylique de formule générale II : 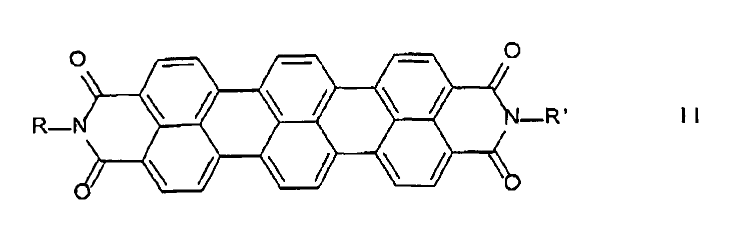 est amené à réagir avec du brome élémentaire en présence d'un halogénohydrocarbure en tant que solvant pour donner du diimide d'acide 1,6,9,14-tétrabromoterrylènetétracarboxylique de formule générale Ia' : 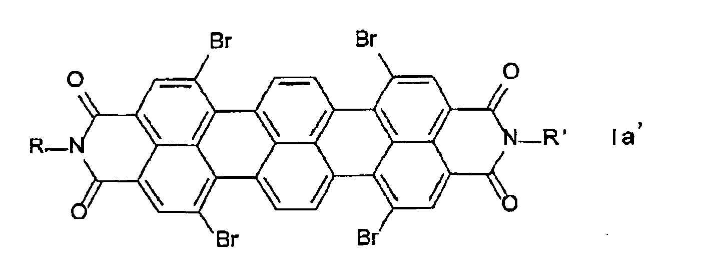 et éventuellement
b) le diimide d'acide tétrabromoterrylènetétracarboxylique Ia' obtenu à l'étape a) est converti en diimide d'acide terrylènetétracarboxylique 1,6,9,14-tétrasubstitué de formule Ia avec X¹ ≠ Br en présence d'un solvant inerte à base d'azote et d'une base avec un alcool ou un thioalcool de formule générale III avec X¹ ≠ Br : 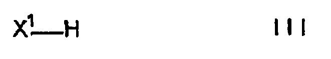 sous réserve que le diimide d'acide N,N'-bis(2,6-diisopropylphényl)-1,6,9,4-tétra(p-tert-octylphényl)terrylène-3,4:11,12-dicarboxylique et le diimide d'acide N-cyclohexyl-N'-(2,6-diisopropylphényl)-1,6,9,14-tétra(p-tert-octylphénoxy)terrylène-3,4:11,12-dicarboxylique soient exclus.

4. Procédé de préparation de diimides d'acide terrylènetétracarboxylique 1,6,9,14-tétrasubstitués de formule générale Ib : 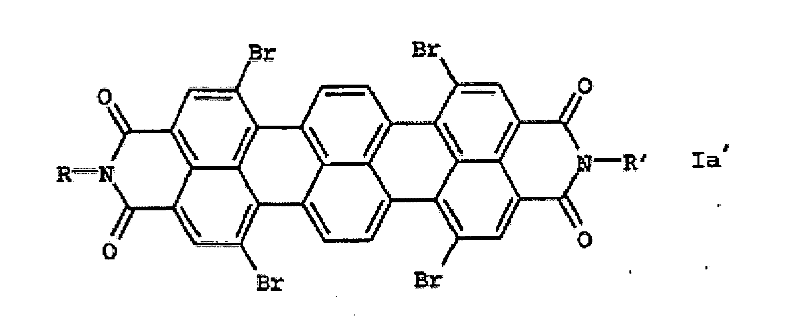 dans laquelle les variables ont la signification suivante :
x² aryloxy, arylthio, hétaryloxy ou hétarylthio, qui peut être substitué respectivement une ou plusieurs fois par alkyle en C₁-C₁₂, alcoxy en C₁-C₁₂, -COOR¹, -SO₃R¹, cyano, -CONHR⁴ et/ou -NHCOR⁴ ;
x¹ brome ; aryloxy, arylthio, hétaryloxy ou hétarylthio, qui peut être substitué respectivement une ou plusieurs fois par alkyle en C₁-C₁₂, alcoxy en C₁-C₁₂, -COOR¹, -SO₃R¹, halogène, hydroxy, carboxy, cyano, -CONHR² et/ou -NHCOR² ;
R, R' indépendamment l'un de l'autre
hydrogène ;
alkyle en C₁-C₃₀, dont la chaîne de carbone peut être interrompue par un ou plusieurs groupements -O-, -S-, -NR¹-, -CO- et/ou -SO₂- et qui peut être substitué une ou plusieurs fois par cyano, alcoxy en C₁-C₆, aryle, qui peut être substitué par alkyle en C₁-C₁₈ ou alcoxy en C₁-C₆, et/ou un radical hétérocyclique pentagonal à heptagonal lié par un atome d'azote, qui peut contenir d'autres hétéroatomes et être aromatique ;
cycloalkyle en C₅-C₈, dont le squelette de carbone peut être interrompu par un ou plusieurs groupements -O-, -S- et/ou -NR¹- et/ou qui peut être substitué une ou plusieurs fois par alkyle en C₁-C₆ ;
aryle ou hétaryle, qui peut être substitué une ou plusieurs fois par alkyle en C₁-C₁₈, alcoxy en C₁-C₆, cyano, -CONHR², -NHCOR² et/ou arylazo ou hétarylazo, qui peut être substitué respectivement par alkyle en C₁-C₁₀, alcoxy en C₁-C₆ ou cyano ;
R¹ hydrogène ou alkyle en C₁-C₆ ;
R² hydrogène ; alkyle en C₁-C₁₈ ; aryle ou hétaryle, qui peut être substitué respectivement par alkyle en C₁-C₆, alcoxy en C₁-C₆, halogène, hydroxy, carboxy ou cyano,
R⁴ alkyle en C₁-C₁₈ ; aryle ou hétaryle, qui peut être substitué respectivement par alkyle en C₁-C₆, alcoxy en C₁-C₆, hydroxy ou cyano,
**caractérisé en ce que** :
a) un diimide d'acide terrylènetétracarboxylique 1,6-disubstitué de formule générale IV : 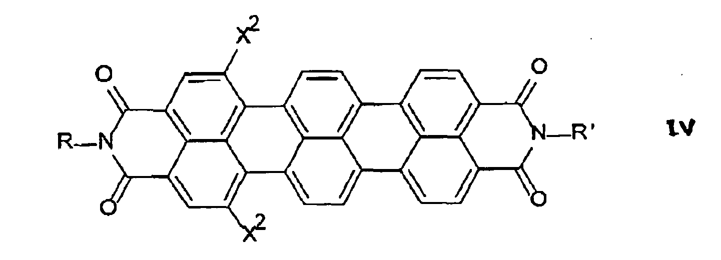 est amené à réagir avec du brome élémentaire en présence d'un halogénohydrocarbure en tant que solvant pour donner du diimide d'acide terrylènetétracarboxylique 9,14-dibromé de formule générale Ib' : 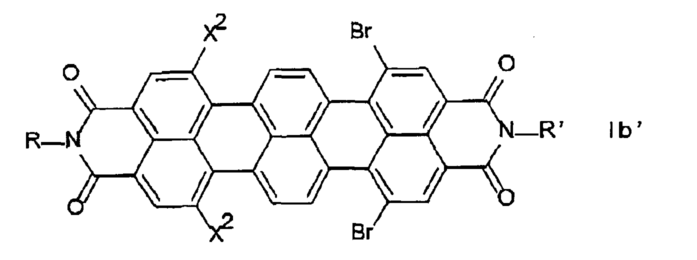 et éventuellement
b) le diimide d'acide terrylènetétracarboxylique 9,14-dibromé Ib' obtenu à l'étape a) est converti en diimide d'acide terrylènetétracarboxylique 1,6,9,14-tétrasubstitué de formule Ib avec X¹ ≠ brome en présence d'un solvant inerte à base d'azote et d'une base avec un alcool ou un thioalcool de formule générale III avec X¹ ≠ brome :
X¹-H III

5. Procédé de préparation de diimides d'acide terrylènetétracarboxylique 1,6,9,14-tétrasubstitués de formule générale Ic : 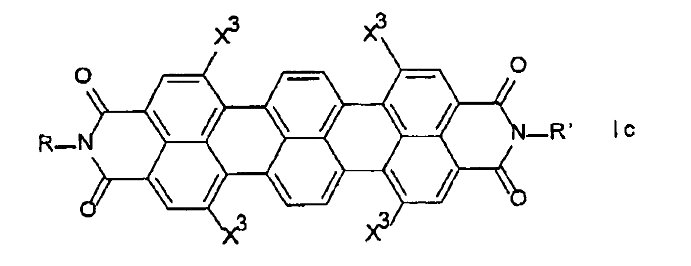 dans laquelle les variables ont la signification suivante :
x³ un radical de formule -L-R³ ; ;
R, R' indépendamment l'un de l'autre
hydrogène ;
alkyle en C₁-C₃₀, dont la chaîne de carbone peut être interrompue par un ou plusieurs groupements -O-, -S-, -NR¹-, -CO- et/ou -SO₂ et qui peut être substitué une ou plusieurs fois par cyano, alcoxy en C₁-C₆, aryle, qui peut être substitué par alkyle en C₁-C₁₈ ou alcoxy en C₁-C₆, et/ou un radical hétérocyclique pentagonal à heptagonal lié par un atome d'azote, qui peut contenir d'autres hétéroatomes et être aromatique ;
cycloalkyle en C₅-C₈, dont le squelette de carbone peut être interrompu par un ou plusieurs groupements -O-, -S- et/ou -NR¹- et/ou qui peut être substitué une ou plusieurs fois par alkyle en C₁-C₆ ;
aryle ou hétaryle, qui peut être substitué une ou plusieurs fois par alkyle en C₁-C₁₈, alcoxy en C₁-C6 , cyano, -CONHR², -NHCOR², et / ou arylazo ou hétarylazo, qui peut être substitué respectivement par alkyle en C₁-C₁₀, alcoxy en C₁-C₆ ou cyano ;
L 1,2-éthylène, 1,2-éthénylène ou 1,2-éthinylène,
R¹ hydrogène ou alkyle en C₁-C₆ ;
R² hydrogène, alkyle en C₁-C₁₈ ; aryle ou hétaryle, qui peut être substitué respectivement par alkyle en C₁-C₆, alcoxy en C₁-C₆, halogène, hydroxy, carboxy ou cyano ;
R³ alkyle en C₁-C₁₈, dont la chaîne de carbone peut être interrompue par un ou plusieurs groupements -O-, -S-, -NR¹-, -CO- et/ou -SO₂- et qui peut être substitué une ou plusieurs fois par -COOR¹, -SO₃R¹, hydroxy, cyano, alcoxy en C₁-C₆, aryle, qui peut être substitué par alkyle en C₁-C₁₈ ou alcoxy en C₁-C₆, et/ou un radical hétérocyclique pentagonal à heptagonal lié par un atome d'azote, qui peut contenir d'autres hétéroatomes et être aromatique,
**caractérisé en ce qu'**un diimide d'acide 1,6,9,14-tétrabromoterrylènetétracarboxylique de formule générale Ia' : 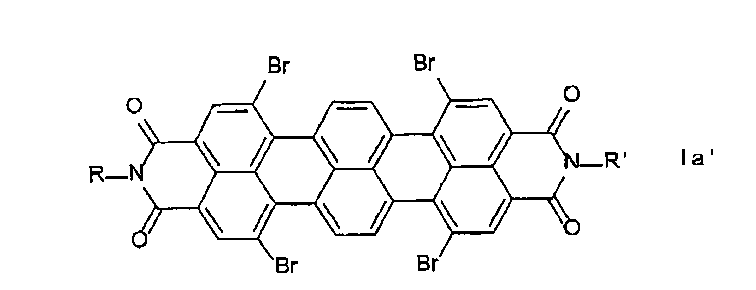 est amené à réagir avec un 1-alcyne de formule générale V :
H-C≡C-R³ V
en présence d'un solvant aprotique, d'un complexe de palladium en tant que catalyseur, d'un sel de cuivre en tant que cocatalyseur et d'une base, et les liaisons insaturées contenues dans les radicaux X³ du diimide d'acide terrylènetétracarboxylique 1,6,9,14-tétrasubstitué obtenu sont éventuellement réduites de manière supplémentaire.

6. Procédé de préparation de diimides d'acide terrylènetétracarboxylique 1,6,9,14-tétrasubstitués de formule générale Id : 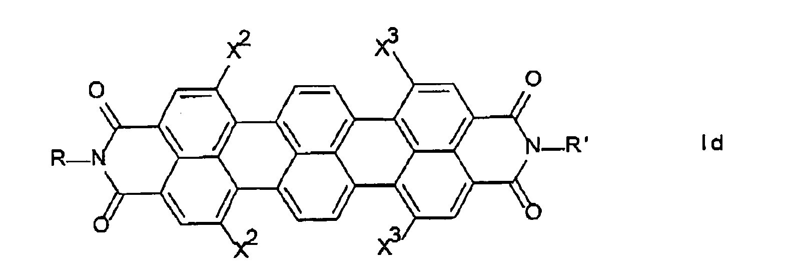 dans laquelle les variables ont la signification suivante :
_{X}² aryloxy, arylthio, hétaryloxy ou hétarylthio, qui peut être substitué respectivement une ou plusieurs fois par alkyle en C₁-C₁₂, alcoxy en C₁-C₁₂, -COOR¹, -SO₃R¹, cyano, -CONHR⁴ et/ou -NHCOR⁴ ;
X³ un radical de formule -L-R³ ;
R, R' identiques ou différents et indépendamment l'un de l'autre
hydrogène ;
alkyle en C₁-C₃₀, dont la chaîne de carbone peut être interrompue par un ou plusieurs groupements -O-, -S-, -NR¹-, -CO- et/ou -SO₂- et qui peut être substitué une ou plusieurs fois par cyano, alcoxy en C₁-C₆, aryle, qui peut être substitué par alkyle en C₁-C₁₈ ou alcoxy en C₁-C₆, et/ou un radical hétérocyclique pentagonal à heptagonal lié par un atome d'azote, qui peut contenir d'autres hétéroatomes et être aromatique ;
cycloalkyle en C₅-C₈, dont le squelette de carbone peut être interrompu par un ou plusieurs groupements -O-, -S- et/ou -NR¹- et/ou qui peut être substitué une ou plusieurs fois par alkyle en C₁-C₆ ;
aryle ou hétaryle, qui peut être substitué une ou plusieurs fois par alkyle en C₁-C₁₈, alcoxy en C₁-C₆, cyano, -CONHR² -NHCOR² et/ou arylazo ou hétarylazo, qui peut être substitué respectivement par alkyle en C₁-C₁₀, alcoxy en C₁-C₆ ou cyano ;
L 1,2-éthylène, 1,2-éthénylène ou 1,2-éthinylène ;
R¹ hydrogène ou alkyle en C₁-C₆ ;
R² hydrogène ; alkyle en C₁-C₁₈ ; aryle ou hétaryle, qui peut être substitué respectivement par alkyle en C₁-C₆, alcoxy en C₁-C₆, halogène, hydroxy, carboxy ou cyano ;
R³ alkyle en C₁-C₁₈, dont la chaîne de carbone peut être interrompue par un ou plusieurs groupements -O-, -S-, -NR¹-, -CO- et/ou -SO₂- et qui peut être substitué une ou plusieurs fois par -COOR¹, -SO₃R¹, hydroxy, cyano, alcoxy en C₁-C₆, aryle, qui peut être substitué par alkyle en C₁-C₁₈ ou alcoxy en C₁-C₆, et/ou un radical hétérocyclique pentagonal à heptagonal lié par un atome d'azote, qui peut contenir d'autres hétéroatomes et être aromatique ;
R⁴ alkyle en C₁-C₁₈ ; aryle ou hétaryle, qui peut être substitué respectivement par alkyle en C₁-C₆, alcoxy en C₁-C₆, halogène, hydroxy, carboxy ou cyano,
**caractérisé en ce qu'**un diimide d'acide terrylènetétracarboxylique 9,14-dibromé de formule générale Ib' : 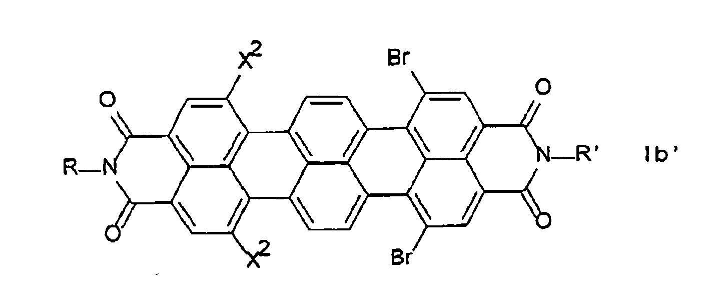 est amené à réagir avec un 1-alcyne de formule générale V :
H-C≡C-R³ V
en présence d'un solvant aprotique, d'un complexe au palladium en tant que catalyseur, d'un sel de cuivre en tant que cocatalyseur et d'une base, et les liaisons insaturées contenues dans les radicaux X³ du diimide d'acide terrylènetétracarboxylique 1,6,9,14-tétrasubstitué obtenu sont éventuellement réduites de manière supplémentaire.

7. Utilisation de diimides d'acide terrylènetétracarboxylique 1,6,9,14-tétrasubstitués de formule I selon la revendication 1 ou 2 pour la coloration de matériaux organiques et minéraux à haut poids moléculaire.

8. Utilisation de diimides d'acide terrylènetétracarboxylique 1,6,9,14-tétrasubstitués de formule I selon la revendication 1 ou 2 en tant qu'auxiliaires de dispersion, additifs de pigment pour des pigments organiques et produits intermédiaires pour la préparation de colorants fluorescents et d'additifs de pigment.

9. Utilisation de diimides d'acide terrylènetétracarboxylique 1,6,9,14-tétrasubstitués de formule I selon la revendication 1 ou 2 pour la préparation de dispersions aqueuses de polymère, colorées ou absorbant et/ou émettant dans la région du proche infrarouge du spectre électromagnétique.

10. Utilisation de diimides d'acide terrylènetétracarboxylique 1,6,9,14-tétrasubstitués de formule I selon la revendication 1 ou 2 en tant qu'émetteurs dans des utilisations électroluminescentes et chimioluminescentes, en tant que composants actifs dans la conversion de fluorescence, dans des miroirs bioluminescents et dans le domaine photovoltaïque et en tant que colorants à laser.
